# EUROPEAN PATENT SPECIFICATION

(11) **EP 1 893 575 B1**
(45) Date of publication and mention of the grant of the patent: **11.01.2017**
(21) Application number: 06808965.5
(22) Date of filing: 14.06.2006
(51) Int. Cl.: C07D 209/30, A61K 31/404

(54) **2-(1H-INDOLYLSULFANYL)-ARYL AMINE DERIVATIVES**
2-(1H-INDOLYLSULFANYL)-ARYL-AMIN-DERIVATE
DERIVES DE 2-(1H-INDOLYLSULFANYL)-ARYL AMINE

(30) Priority: 17.06.2005 US 692009 P
(43) Date of publication of application: 05.03.2008
(73) Proprietor: H. Lundbeck A/S, 2500 Valby (DK)
(72) Inventor: KEHLER, Jan, 2800 Kgs. Lyngby (DK); JUHL, Karsten, 2670 Greve (DK); NORGAARD, Morten Bang, 2800 Lyngby (DK)
(74) Representative: H. Lundbeck A/S
(86) International application number: PCT/IB2006/002785
(87) International publication number: WO 2006/134499

(56) References cited:
- WO-A-97/17352
- WO-A-03/029232
- WO-A-2005/061455
- ANTHONY A. JACKSON, DAVID N. JOHNSTON, PATRICK V. R. SHANNON: "Electrophilic Substitution in Indoles Part 16: The Formation of Indolobenzothiazines and Indolobenzothiazepines by Intramolecular Cyclisation of (o-Nitrophenylthio)indoles" J. CHEM. RES. MINIPRINT, vol. 9, 1988, pages 2017-2063, XP009077459
- RINO RAGNO, MARINO ARTICO, GABRIELLA DE MARTINO, GIUSEPPE LA REGINA, ANTONIO COLUCCIA ET AL: "Docking and 3-D QSAR Studies on Indolyl Aryl Sulfones. Binding Mode Exploration at the HIV-1 Reverse Transcriptase Non-Nucleoside Binding Site and Design of Highly Active N-(2-Hydroxyethyl)carboxamide and N-(2-hydroxyethyl)carbohydrazide Derivatives" J. MED. CHEM., vol. 48, no. 1, 2005, pages 213-223, XP002415706
- ROMANO SILVESTRI, GABRIELLA DE MARTINO, GIUSEPPE LA REGINA, MARINO ARTICO, SILVIO MASSA ET AL: "Novel Indolyl Aryl Sulfones Active against HIV-1 Carrying NNRTI Resistance Mutations: Synthesis and SAR Studies" J. MED. CHEM., vol. 46, 2003, pages 2482-2493, XP002415707

## Description

### FIELD OF THE INVENTION

The present invention relates to compounds of formula IV and the medical use thereof e.g. in the treatment of affective disorders, pain disorders, attention deficit hyperactivity disorder (ADHD) and stress urinary incontinence.

### BACKGROUND OF THE INVENTION

The majority of currently available antidepressants can be classified in 3 classes:
1) monoamine oxidase inhibitors (MAOIs),
2) biogenic amine neurotransmitter [serotonin (5-HT), norepinephrine (NE) and dopamine (DA)] transporter reuptake blockers, and
3) modulators, especially blockers of one or more of the 5-HT and/or NE receptors.

Since depression is associated with a relative deficiency of the biogenic amines, the use of 5-HT and/or NE-receptor blockers (i.e. 5-HT and or NE-antagonist's) have not proven very successful in the treatment of depression and anxiety and the preferred and currently most efficacious treatments are based on the enhancement of 5-HT and/or NE neurotransmission by blocking their reuptake back from the synaptic cleft (Slattery, D.A. et al., "The evolution of antidepressant mechanisms", fundamental and Clinical pharmacology, 2004, 18, 1-21; Schloss, P. et al, "new insights into the mechanism of antidepressant therapy", Pharmacology and therapeutics, 2004, 102, 47-60).

Selective serotonin reuptake inhibitors (hereinafter referred to as SSRIs) have become first choice therapeutics in the treatment of depression, certain forms of anxiety and social phobias, because they generally are effective, well tolerated and have a favourable safety profile compared to the classic tricyclic antidepressants. Drugs claimed to be SSRIs are for example fluoxetine, sertraline and paroxetine.

However, clinical studies on depression indicate that non-response to the known SSRIs is substantial, up to 30%. Another, often neglected, factor in the treatment of depression is the delay in the onset of the therapeutic effect of the SSRIs. Sometimes symptoms even worsen during the first weeks of treatment. Furthermore, sexual dysfunction is generally a side effect common to SSRIs. Accordingly, there is a desire for the development of compounds capable of improving the treatment of depression and other diseases related to malfunctioning of serotonin.

Dual re-uptake inhibitors providing the combined effect of 5-HT reuptake inhibition and NE (norepinephrine is also named noradrenaline, NA) reuptake inhibition on depression is explored in clinical studies of compounds such as Duloxetine (Wong, "Duloxetine (LY-248686): an inhibitor of serotonin and noradrenaline uptake and an antidepressant drug candidate", Expert Opinion on Investigational Drugs, 1998, 7, 10, 1691-1699) and Venlafaxine (Khan-A et al, 30 "Venlafaxine in depressed outpatients", Psychopharmacology Bulletin, 1991, 27, 141-144). Compounds having such dual effect are also named SNRIs, "serotonin and noradrenaline reuptake inhibitors", or NSRIs, "noradrenaline and serotonin reuptake inhibitors".

Since treatment with the selective NE reuptake inhibitor reboxetine has been shown to stimulate 5-HT neurons and mediate the release of 5-HT in the brain (Svensson,T. et al, J. Neural. Transmission, 2004, 111, 127) there might be a synergistic advantage using SNRI's in the treatment of depression or anxiety.

The use of SNRI's have been shown in clinical studies to have a beneficial effect on pain (e.g. Fibromyalgia syndrome, overall pain, back pain, shoulder pain, headache, pain while awake and during daily activities) and especially pain associated with depression (Berk, M. Expert Rev. Neurotherapeutics 2003, 3, 47-451; Fishbain, D.A., et al. "Evidence-based data from animal and human experimental studies on pain relief with antidepressants: A structured review" Pain Medicine 2000 1:310-316).

SNRI's have also been shown in clinical studies to have a beneficial effect in attention deficit hyperactivity disorder (ADHD) (N. M. Mukaddes; Venlafaxine in attention deficit hyperactivity disorder, European Neuropsychopharmacology, Volume 12, Supplement 3, October 2002, Page 421).

Furthermore, SNRI's have been shown to be effective for the treatment of stress urinary incontinence (Dmochowski R.R. et al. "Duloxetine versus placebo for the treatment of North American women with stress urinary incontinence", Journal of Urology 2003, 170:4, 1259-1263.)

Naranjo, C. et al. " The role of the brain reward system in depression" Prog. Neuro-Psychopharmacol. Biol. Psychiatry 2001, 25, 781- 823 discloses clinical and preclinical findings of links between lack of extra cellular dopamine in the mesocorticolimbic system and anhedonia, which is one of the main symptoms of depression.

Wellbutrin (bupropion) which has DA re-uptake activity in vitro and in vivo, shows antidepressant efficacy. Other combination studies have indicated that addition of some affinity at the DA uptake site may have some clinical benefit (Nelson, J. C. J. Clin. Psychiatry 1998, 59, 65; Masand, P. S. et al. Depression Anxiety 1998, 7, 89; Bodkin, J. A et al. J. Clin. Psychiatry 1997, 58, 137).

Axford L. et al. (2003, Bioorganic & Medical Chemistry Letters, 13, 3277-3280: "Bicyclo[2.2.1.]heptanes as novel triple re-uptake inhibitors for the treatment of depression") describe the development of triple 5-HT, NE and DA re-uptake inhibitors for treatment of depression. The combination of an SSRI and a norepinephrine and dopamine reuptake inhibitor, has been shown to have better efficacy in SSRI-non-responders (Lam R. W. et al. "Citalopram and Bupropion-SR: Combining Versus Switching in Patients With Treatment-Resistant Depression." J. Clin. Psychiatry 2004, 65, 337-340).

There is clinical evidence suggesting the combination of an SSRI and a norepinephrine and dopamine reuptake inhibitor induces less sexual dysfunction, than treatment with SSRI's alone (Kennedy S. H. et al. " Combining Bupropion SR With Venlafaxine, Paroxetine, or Duloxetine: A Preliminary Report on Pharmacokinetic, Therapeutic, and Sexual Dysfunction Effects" J. Clin. Psychiatry 2002, 63, 181-186).

Diphenyl sulphides of formula I and variations thereof have been disclosed as serotonin re-uptake inhibitors and have been suggested for use in treatment of depression, cf. e.g. WO03029232(A1).

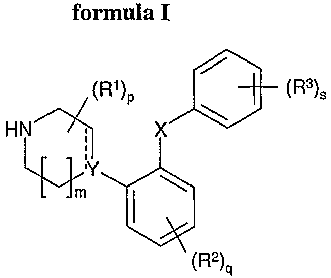

Diphenyl sulphides of formula II and variations thereof have been disclosed as serotonin re-uptake inhibitors and have been suggested for use in treatment of depression, cf. e.g. US 5095039, US 4056632, EP 396827 A1 and WO 9312080. EP 402097 describes halogen substituted diphenylsulfides claimed to be selective serotonin inhibitors for treatment of depression. Likewise WO 9717325 disclose derivatives of N,N-dimethyl-2-(arylthio)benzylamine claimed to be selective serotonin transport inhibitors and suggest their use as antidepressants. J. Jilek et al., , Collect. Czeck Chem. Commun. 1989, 54, 3294-3338 also discloses various derivatives of diphenyl sulphides, "phenyl-thio-benzylamines" as antidepressants. Furthermore, diphenyl sulphides are also disclosed in US 3803143 and claimed useful as antidepressant.

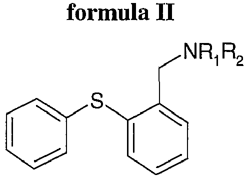

K. Sindelar et al., (K. Sindelar et al. Collect. Czeck Chem. Commun. 1991, 56, 449-458) disclose compounds of formula III with test for selectivity as 5-HT re-uptake inhibitor and NA re-uptake inhibitor, respectively, for use as antidepressants.

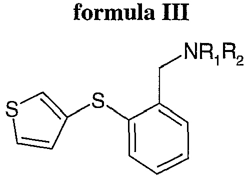

The above-mentioned references do not disclose compounds comprising an indole group like the indolyl-sulfanyl arylamines of the present invention.

The present invention provides 2-(1H-indolylsulfanyl)-aryl amine derivatives of formula IV which are serotonin reuptake inhibitors. A particular aspect of the invention provides compounds possessing the combined effect of serotonin reuptake inhibition and norepinephrine reuptake inhibition. Another particular aspect of the invention provides compounds possessing the combined effect of serotonin reuptake inhibition and dopamine reuptake inhibition. Furthermore, some of the compounds are also triple 5-HT, NE and DA re-uptake inhibitors. 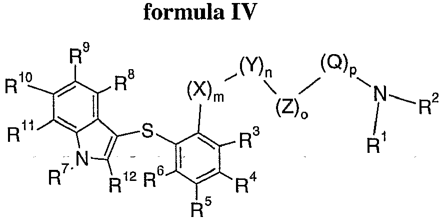 wherein X, Y, Z, Q, m, n, o, p and R¹-R¹² are as defined below.

### SUMMARY OF THE INVENTION

One object of the invention is the provision of compounds, which are serotonin reuptake inhibitors. Another object of the invention is the provision of compounds, which are both serotonin reuptake inhibitors and noradrenaline reuptake inhibitors. Yet another object of the invention is the provision of compounds, which are both serotonin reuptake inhibitors and dopamine reuptake inhibitors. Yet another object of the invention is the provision of compounds, which are serotonin reuptake inhibitors, noradrenaline reuptake inhibitors and dopamine reuptake inhibitors.

The compounds of the invention are substituted indole derivatives of the general formula IV as the free base or salts thereof.

The invention provides a compound according to the above for use as a medicament.

The invention provides a pharmaceutical composition comprising a compound according to the above and at least one pharmaceutically acceptable carrier or diluent.

The invention provides the use of a compound according to the above for the preparation of a pharmaceutical composition for the treatment of affective disorders, pain disorders, ADHD and stress urinary incontinence.

The invention furthermore concerns the use of a compound according to the above in a method of treatment of affective disorders, pain disorders, ADHD and stress urinary incontinence.

### DEFINITION OF SUBSTITUENTS

The term heteroatom refers to a nitrogen, oxygen or sulphur atom.

Halo means halogen. Halogen means fluoro, chloro, bromo or iodo.

The expression "C₁₋₆-alk(en/yn)yl" means a C₁₋₆-alkyl, a C₂₋₆-alkenyl or a C₂₋₆-alkynyl group. The term "C₁₋₆-alkyl" refers to a branched or unbranched alkyl group having from one to six carbon atoms inclusive, including but not limited to methyl, ethyl, 1-propyl, 2-propyl, 1-butyl, 2-butyl, 2-methyl-2-propyl and 2-methyl-1-propyl. The term "C₂₋₆-alkenyl" refers to a branched or unbranched alkenyl group having from two to six carbon atoms, including one double bond, including but not limited to ethenyl, propenyl, and butenyl. The term "C₂₋₆-alkynyl" refers to a branched or unbranched alkynyl group having from two to six carbon atoms, including one triple bond, including but not limited to ethynyl, propynyl and butynyl.

The expression "C₃₋₈-cycloalk(en)yl" means a C₃₋₈-cycloalkyl or a C₃₋₈-cycloalkenyl group. The term "C₃₋₈-cycloalkyl" designates a monocyclic or bicyclic carbocycle having three to eight C-atoms, including but not limited to cyclopropyl, cyclopentyl, and cyclohexyl. The term "C₃₋₈-cycloalkenyl" designates a monocyclic or bicyclic carbocycle having three to eight C-atoms and one double bond, including but not limited to cyclopropenyl, cyclopentenyl and cyclohexenyl.

In the expression "C₃₋₈-cycloalk(en)yl-C₁₋₆-alk(en/yn)yl", "C₁₋₆-alk(en/yn)ylamino", "di-(C₁₋₆-alk(en/yn)yl)amino", "C₁₋₆-alk(en/yn)ylcarbonyl", "C₁₋₆-alk(en/yn)ylaminocarbonyl", "di-(C₁₋₆-alk(en)yl)aminocarbonyl", "C₁₋₆-alk(en/yn)yloxy", "C₁₋₆-alk(en/yn)ylsulfanyl", "halo-C₁₋₆-alk(en/yn)yl", "halo-C₁₋₆-alk(en/yn)ylsulfonyl", "halo-C₁₋₆-alk(en/yn)ylsulfanyl" and "C₁₋₆-alk(en/yn)ylsulfonyl" the terms "amino", "C₃₋₈-cycloalk(en)yl", "C₁₋₆-alk(en/yn)yl", "C₁₋₆-alk(en)yl" and "halo" are as defined above.

The term "aminocarbonyl" designates NH₂-C=O which is attached to the remainder of the molecule via the carbon atom.

The term "R¹ and R² together with the nitrogen form a 4-7 membered ring containing zero or one double bond, optionally said ring in addition to said nitrogen comprises one further heteroatom selected from oxygen and sulphur" refers to such ring systems wherein a ring is formed by the nitrogen to which R¹ and R² are attached and 3-6 atoms selected from 2-6 carbonatoms and 0-1 heteroatoms selected from sulphur and oxygen, said ring contains zero or one double bond. Examples of rings formed by R¹, R² and the nitrogen to which they are attached are pyrrolidine, piperidine, morpholine and thiomorpholine..

### DESCRIPTION OF THE INVENTION

The present invention relates to a compound represented by the general formula IV 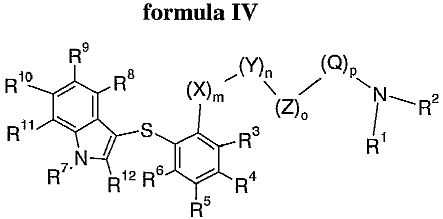 wherein
R¹-R² are independently selected from hydrogen, C₁₋₆-alk(en/yn)yl, C₃₋₈-cycloalk(en)yl, and C₃₋₈-cycloalk(en)yl-C₁₋₆-alk(en/yn)yl; or R¹ and R² together with the nitrogen form a 4-7 membered ring containing zero or one double bond, optionally said ring in addition to said nitrogen comprises one further heteroatom selected from oxygen and sulphur;
R³-R⁶ and R⁸-R¹² are independently selected from hydrogen, halogen, cyano, nitro, C₁₋₆-alk(en/yn)yl, C₃₋₈-cycloalk(en)yl, C₃₋₈-cycloalk(en)yl-C₁₋₆-alk(en/yn)yl, amino, C₁₋₆-alk(en/yn)ylamino, di-(C₁₋₆-alk(en/yn)yl)amino, C₁₋₆-alk(en/yn)ylcarbonyl, aminocarbonyl, C₁₋₆-alk(en/yn)ylaminocarbonyl, di-(C₁₋₆-alk(en/yn)yl)aminocarbonyl, hydroxy, C₁₋₆-alk(en/yn)yloxy, C₁₋₆-alk(en/yn)ylsulfanyl, halo-C₁₋₆-alk(en/yn)yl, halo-C₁₋₆-alk(en/yn)ylsulfonyl, halo-C₁₋₆-alk(en/yn)ylsulfanyl and C₁₋₆-alk(en/yn)ylsulfonyl;
R⁷ is selected from hydrogen, C₁₋₆-alk(en/yn)yl, C₃₋₈-cycloalk(en)yl and C₃₋₈-cycloalk(en)yl-C₁₋₆-alk(en/yn)yl;
X is selected from the group consisting of CH₂, CHR¹³ or CR¹⁴R¹⁵;
Y is selected from the group consisting of CH₂, CHR¹⁶ and CR¹⁷R¹⁸;
Z is selected from the group consisting of CH₂, CHR¹⁹ and CR²⁰R²¹; and
Q is selected from the group consisting of CH₂, CHR²² and CR²³R²⁴;
m, n, o and p are independently 0 or 1,wherein m+n+o+p equals to 1, 2, 3 or 4, with the proviso that when m+ n + o + p equals to 1
   then none of X, Y, Z and Q are CH₂;
wherein R¹³- R²⁴ are independently selected from the group consisting of C₁₋₆-alk(en/yn)yl, C₃₋₈-cycloalk(en)yl and C₃₋₈-cycloalk(en)yl-C₁₋₆-alk(en/yn)yl;
as the free base or a salt thereof.

In one embodiment of the compound of formula IV, R¹ and R² are independently selected from the group consisting of C₃₋₈-cycloalk(en)yl and C₃₋₈-cycloalk(en)yl-C₁₋₆-alk(en/yn)yl. In a further embodiment of the compound of formula IV, R¹ and R² are independently selected from the group consisting of hydrogen and C₁₋₆-alk(en/yn)yl; or R¹ and R² together with the nitrogen form a 4-7 membered ring containing zero or one double bond, optionally said ring in addition to said nitrogen comprises one further heteroatom selected from oxygen and sulphur.

To further illustrate without limiting the invention an embodiment of R¹ is hydrogen; another embodiment of R¹ is C₁₋₆-alk(en/yn)yl such as methyl.

To further illustrate without limiting the invention an embodiment of R² is hydrogen; another embodiment of R² is C₁₋₆-alk(en/yn)yl such as methyl.

To further illustrate without limiting the invention, an embodiment of the compound of formula IV concerns such compounds wherein R¹ and R² together with the nitrogen form a 4-7 membered ring containing zero or one double bond, optionally said ring in addition to said nitrogen comprises one further heteroatom selected from oxygen and sulphur. In one embodiment said 4-7 membered ring does not contain any double bond; in another embodiment said 4-7 membered ring does contain one double bond. In one embodiment the only heteroatom contained in said 4-7 membered ring is the nitrogen to which R¹ and R² are attached. In another embodiment said 4-7 membered ring contains one heteroatom in addition to the nitrogen to which R¹ and R² are attached; in a further embodiment said heteroatom is sulphur; in a further embodiment said heteroatom is oxygen. Typically said 4-7 membered ring is selected from the group consisting of morpholine and thiomorpholine.

In a further embodiment of the compound of formula IV, R³-R⁶ and R⁸-R¹² are independently selected from the group consisting of C₁₋₆-alk(en/yn)ylcarbonyl, aminocarbonyl, C₁₋₆-alk(en/yn)ylaminocarbonyl, di-(C₁₋₆-alk(en/yn)yl)aminocarbonyl and hydroxy.
In a further embodiment of the compound of formula IV, R³-R⁶ and R⁸-R¹² are independently selected from the group consisting of hydrogen, halogen, cyano, nitro, C₁₋₆-alk(en/yn)yl, C₃₋₈-cycloalk(en)yl, C₃₋₈-cycloalk(en)yl-C₁₋₆-alk(en/yn)yl, amino, C₁₋₆-alk(en/yn)ylamino, amino, C₁₋₆-alk(en/yn)yloxy, C₁₋₆-alk(en/yn)ylsulfanyl, halo-C₁₋₆-alk(en/yn)yl, halo-C₁₋₆-alk(en/yn)ylsulfonyl, halo-C₁₋₆-alk(en/yn)ylsulfanyl and C₁₋₆-alk(en/yn)ylsulfonyl
In a further embodiment of the compound of formula IV, R³-R⁶ and R⁸-R¹² are independently selected from the group consisting of hydrogen, halogen, nitro, C₁₋₆-alk(en/yn)yl, di-(C₁₋₆-alk(en/yn)yl)amino, C₁₋₆-alk(en/yn)yloxy and C₁₋₆-alk(en/yn)ylsulfonyl.
In a further embodiment of the compound of formula IV, R³-R⁶ and R⁸-R¹² are independently selected from the group consisting of hydrogen, halogen, C₁₋₆-alk(en/yn)yl, and C₁₋₆-alk(en/yn)yloxy.

In an embodiment of the compound of formula IV, R³-R⁶ are independently selected from the group consisting of nitro, amino, C₁₋₆-alk(en/yn)ylamino, di-(C₁₋₆-alk(en/yn)yl)amino, C₁₋₆-alk(en/yn)ylcarbonyl, aminocarbonyl, C₁₋₆-alk(en/yn)ylaminocarbonyl, di-(C₁₋₆-alk(en/yn)yl)aminocarbonyl, hydroxy, halo-C₁₋₆-alk(en/yn)ylsulfonyl and C₁₋₆-alk(en/yn)ylsulfonyl.

In a further embodiment of the compound of formula IV, R³-R⁶ are independently selected from the group consisting of hydrogen, halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, C₁₋₆-alk(en/yn)yloxy, C₁₋₆-alk(en/yn)ylsulfanyl and halo-C₁₋₆-alk(en/yn)ylsulfanyl. Typically, R³-R⁶ are independently selected from the group consisting of hydrogen, halogen and C₁₋₆-alk(en/yn)yloxy.
To further illustrate without limiting the invention an embodiment of R³ is hydrogen. Typically, R⁴ is selected from the group consisting of hydrogen, halogen and C₁₋₆-alk(en/yn)yloxy. To further illustrate without limiting the invention an embodiment of R⁴ is hydrogen; another embodiment of R⁴ is halogen such as chloro or fluoro; another embodiment of R⁴ is C₁₋₆-alk(en/yn)yloxy such as methoxy.
Typically, R⁵ is selected from the group consisting of hydrogen and C₁₋₆-alk(en/yn)yloxy. To further illustrate without limiting the invention an embodiment of R⁵ is hydrogen; another embodiment of R⁵ is C₁₋₆-alk(en/yn)yloxy such as methoxy.
To further illustrate without limiting the invention an embodiment of R⁶ is hydrogen.

In a further embodiment of the compound of formula IV, R⁸-R¹² are independently selected from the group consisting of C₁₋₆-alk(en/yn)ylcarbonyl, aminocarbonyl, C₁₋₆-alk(en/yn)ylaminocarbonyl, di-(C₁₋₆-alk(en/yn)yl)aminocarbonyl and hydroxy.
In a further embodiment of the compound of formula IV, R⁸-R¹² are independently selected from the group consisting of hydrogen, halogen, cyano, nitro, C₁₋₆-alk(en/yn)yl, C₃₋₈-cycloalk(en)yl, C₃₋₈-cycloalk(en)yl-C₁₋₆-alk(en/yn)yl, amino, C₁₋₆-alk(en/yn)ylamino, di-(C₁₋₆-alk(en/yn)yl)amino, C₁₋₆-alk(en/yn)yloxy, C₁₋₆-alk(en/yn)ylsulfanyl, halo-C₁₋₆-alk(en/yn)yl, halo-C₁₋₆-alk(en/yn)ylsulfonyl, halo-C₁₋₆-alk(en/yn)ylsulfanyl and C₁₋₆-alk(en/yn)ylsulfonyl;
Typically, R⁸-R¹² are independently selected from the group consisting of hydrogen, halogen, nitro, C₁₋₆-alk(en/yn)yl, di-(C₁₋₆-alk(en/yn)yl)amino, C₁₋₆-alk(en/yn)yloxy and C₁₋₆-alk(en/yn)ylsulfonyl.
In a further embodiment of the compound of formula IV, R⁸-R¹² are independently selected from the group consisting of hydrogen, halogen, C₁₋₆-alk(en/yn)yl and C₁₋₆-alk(en/yn)yloxy. Typically, R⁸ is selected from the group consisting of hydrogen, halogen and C₁₋₆-alk(en/yn)yloxy. To further illustrate without limiting the invention an embodiment of R⁸ is hydrogen; another embodiment of R⁸ is halogen such as chloro or fluoro; another embodiment of R⁸ is C₁₋₆-alk(en/yn)yloxy such as methoxy.
Typically, R⁹ is selected from the group consisting of hydrogen, halogen, C₁₋₆-alk(en/yn)yl and di-(C₁₋₆-alk(en/yn)yl)amino. To further illustrate without limiting the invention an embodiment of R⁹ is hydrogen; another embodiment of R⁹ is halogen such as chloro or fluoro; another embodiment of R⁹ is C₁₋₆-alk(en/yn)yl such as methyl; another embodiment of R⁹ is di-(C₁₋₆-alk(en/yn)yl)amino such as dimethylamino.
Typically, R¹⁰ is selected from the group consisting of hydrogen, halogen and C₁₋₆-alk(en/yn)ylsulfonyl. To further illustrate without limiting the invention an embodiment of R¹⁰ is hydrogen; another embodiment of R¹⁰ is halogen such as fluoro; another embodiment of R¹⁰ is C₁₋₆-alk(en/yn)ylsulfonyl such as methylsulfonyl.
Typically, R¹¹ is selected from the group consisting of hydrogen, halogen, nitro and C₁₋₆-alk(en/yn)yloxy. To further illustrate without limiting the invention an embodiment of R¹¹ is hydrogen; another embodiment of R¹¹ is halogen such as chloro or fluoro; another embodiment of R¹¹ is nitro; another embodiment of R¹¹ is C₁₋₆-alk(en/yn)yloxy such as methoxy.
Typically, R¹² is selected from the group consisting of hydrogen and C₁₋₆-alk(en/yn)yl. To further illustrate without limiting the invention an-embodiment of R¹² is hydrogen; another embodiment of R¹² is C₁₋₆-alk(en/yn)yl such as methyl.

In a further embodiment of the compound of formula IV, R⁷ is selected from the group consisting of C₃₋₈-cycloalk(en)yl and C₃₋₈-cycloalk(en)yl-C₁₋₆-alk(en/yn)yl.
Typically, R⁷ is selected from the group consisting of hydrogen and C₁₋₆-alk(en/yn)yl. To further illustrate without limiting the invention an embodiment of R⁷ is hydrogen; another embodiment of R¹² is C₁₋₆-alk(en/yn)yl such as methyl.

In a further embodiment of the compound of formula IV, X is selected from the group consisting of CHR¹³ and CR¹⁴R¹⁵, Y is selected from the group consisting of CHR¹⁶ and CR¹⁷R¹⁸, Z is selected from the group consisting of CHR¹⁹ and CR²⁰R²¹, and
Q is selected from the group consisting of CHR²² and CR²³R²⁴.
In a further embodiment of the compound of formula IV, X, Y, Z and Q are CH₂.

In a further embodiment of the compound of formula IV, m+ n + o + p equals to 1, 2, 3 or 4; in another embodiment of formula IV, m+ n + o + p equals to 1; in another embodiment of formula IV, m+ n + o + p equals to 2; in another embodiment of formula IV, m+ n + o + p equals to 3; in another embodiment of formula IV, m+ n + o + p equals to 4.

In a further embodiment of the compound of formula IV said compound is selected from the following list of compounds:

| **Compound No** | **Name** |
|---|---|
| 1 | {2-[5-Fluoro-2-(1H-indol-3-ylsulfanyl)-phenyl]-ethyl}-methyl-amine |
| 2 | {2-[2-(6-Fluoro-1H-indol-3-ylsulfanyl)-phenyl]-ethyl}-methyl-amine |
| 3 | {2-[2-(5-Fluoro-1H-indol-3-ylsulfanyl)-phenyl]-ethyl}-methyl-amine |
| 4 | {2-[2-(4-Fluoro-1H-indol-3-ylsulfanyl)-phenyl]-ethyl}-methyl-amine |
| 5 | {2-[2-(7-Fluoro-1H-indol-3-ylsulfanyl)-phenyl]-ethyl}-methyl-amine |
| 6 | {2-[2-(7-Methoxy-1H-indol-3-ylsulfanyl)-phenyl]-ethyl}-methyl-amine |
| 7 | {2-[2-(5-Fluoro-2-methyl-1H-indol-3-ylsulfanyl)-phenyl]-ethyl}-methylamine |
| 8 | {2-[2-(5-Chloro-1H-indol-3-ylsulfanyl)-phenyl]-ethyl}-methyl-amine |
| 9 | {2-[2-(4-Chloro-1H-indol-3-ylsulfanyl)-phenyl]-ethyl}-methyl-amine |
| 10 | {2-[2-(7-Chloro-1H-indol-3-ylsulfanyl)-phenyl]-ethyl}-methyl-amine |
| 11 | {2-[2-(1H-Indol-3-ylsulfanyl)-phenyl]-ethyl}-methyl-amine |
| 12 | {2-[2-(1-Methyl-indol-3-ylsulfanyl)-phenyl]-ethyl}-methyl-amine |
| 13 | {2-[5-Chloro-2-(1H-indol-3-ylsulfanyl)-phenyl]-ethyl}-methyl-amine |
| 14 | {2-[5-Chloro-2-(6-fluoro-1H-indol-3-ylsulfanyl)-phenyl]-ethyl}-methyl-amine |
| 15 | {2-[5-Chloro-2-(4-chloro-1H-indol-3-ylsulfanyl)-phenyl]-ethyl}-methylamine |
| 16 | {2-[5-Fluoro-2-(6-fluoro-1H-indol-3-ylsulfanyl)-phenyl]-ethyl}-methyl-amine |
| 17 | (2-(2-(4-Chloro-1H-indol-3-ylsulfanyl)-5-fluoro-phenyl)-ethyl)-methyl-amine |
| 18 | {2-[2-(6-Fluoro-1H-indol-3-ylsulfanyl)-4,5-dimethoxy-phenyl]-ethyl}-methyl-amine |
| 19 | {2-[2-(4-Chloro-1H-indol-3-ylsulfanyl)-4,5-dimethoxy-phenyl]-ethyl}-methyl-amine |
| 20 | {2-[2-(1H-Indol-3-ylsulfanyl)-4,5-dimethoxy-phenyl]-ethyl}-methyl-amine |
| 21 | {4-[2-(1H-Indol-3-ylsulfanyl)-phenyl]-butyl}-methyl-amine |
| 22 | {4-[2-(4-Methoxy-1H-indol-3-ylsulfanyl)-phenyl]-butyl}-methyl-amine |
| 23 | {4-[2-(4-Chloro-1H-indol-3-ylsulfanyl)-phenyl]-butyl}-methyl-amine |
| 24 | Methyl-{4-[2-(1-methyl-1H-indol-3-ylsulfanyl)-phenyl]-butyl}-amine |
| 25 | {3-[2-(4-Chloro-1H-indol-3-ylsulfanyl)-phenyl]-propyl}-methyl-amine |
| 26 | {3-[2-(4-Methoxy-1H-indol-3-ylsulfanyl)-phenyl]-propyl}-methyl-amine |
| 27 | Dimethyl-{3-[2-(3-methylamino-propyl)-phenylsulfanyl]-1H-indol-5-yl}-amine |
| 28 | Methyl-{3-[2-(7-nitro-1H-indol-3-ylsulfanyl)-phenyl]-propyl}-amine |
| 29 | {3-[2-(6-Methanesulfonyl-1H-indol-3-ylsulfanyl)-phenyl]-propyl}-methyl-amine |
| 30 | {3-[2-(1H-Indol-3-ylsulfanyl)-phenyl]-propyl}-methyl-amine |
| 31 | {3-[2-(6-Fluoro-1H-indol-3-ylsulfanyl)-phenyl]-propyl}-methyl-amine |
| 32 | Methyl-{3-[2-(1-methyl-1H-indol-3-ylsulfanyl)-phenyl]-propyl}-amine |
| 33 | Methyl-{3-[2-(5-methyl-1H-indol-3-ylsulfanyl)-phenyl]-propyl}-amine |
| 34 | 2-[2-(1H-Indol-3-ylsulfanyl)-phenyl]-ethylamine |
| 35 | {2-[2-(1H-Indol-3-ylsulfanyl)-phenyl]-ethyl}-dimethyl-amine |
| 36 | 3-[2-(2-Morpholin-4-yl-ethyl)-phenylsulfanyl]-1H-indole |
| 37 | 3-[2-(2-Thiomorpholin-4-yl-ethyl)-phenylsulfanyl]-1H-indole |

as the free base or a salt thereof. Each of these compounds is considered a specific embodiment and may be subjected to individual claims.

The present invention comprises the free base and salts of the compounds of the invention, typically, pharmaceutically acceptable salts. The salts of the invention include acid addition salts, metal salts, ammonium and alkylated ammonium salts.

The salts of the invention are preferably acid addition salts. The acid addition salts of the invention are preferably pharmaceutically acceptable salts of the compounds of the invention formed with non-toxic acids. Acid addition salts include salts of inorganic acids as well as organic acids. Examples of suitable inorganic acids include hydrochloric, hydrobromic, hydroiodic, phosphoric, sulfuric, sulfamic, nitric acids and the like. Examples of suitable organic acids include formic, acetic, trichloroacetic, trifluoroacetic, propionic, benzoic, cinnamic, citric, fumaric, glycolic, itaconic, lactic, methanesulfonic, maleic, malic, malonic, mandelic, oxalic, picric, pyruvic, salicylic, succinic, methane sulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, theophylline acetic acids, as well as the 8-alotheophyllines, for example 8-bromotheophylline and the like. Further examples of pharmaceutical acceptable inorganic or organic acid addition salts include the pharmaceutically acceptable salts listed in J. Pharm. Sci. 1977, 66, 2, which is incorporated herein by reference.

Also intended as acid addition salts are the hydrates, which the present compounds are able to form.

Examples of metal salts include lithium, sodium, potassium, magnesium salts and the like.

Examples of ammonium and alkylated ammonium salts include ammonium, methyl-, dimethyl-, trimethyl-, ethyl-, hydroxyethyl-, diethyl-, n-butyl-, sec-butyl-, tert-butyl-, tetramethylammonium salts and the like.

Further, the compounds of this invention may exist in unsolvated as well as in solvated forms with pharmaceutically acceptable solvents such as water, ethanol and the like. In general, the solvated forms are considered equivalent to the unsolvated forms for the purposes of this invention.

The compounds of the present invention may have one or more asymmetric centre and it is intended that any optical isomers (i.e. enantiomers or diastereomers), as separated, pure or partially purified optical isomers and any mixtures thereof including racemic mixtures, i.e. a mixture of stereoisomeres, are included within the scope of the invention.

Racemic forms can be resolved into the optical antipodes by known methods, for example, by separation of diastereomeric salts thereof with an optically active acid, and liberating the optically active amine compound by treatment with a base. Another method for resolving racemates into the optical antipodes is based upon chromatography on an optically active matrix. Racemic compounds of the present invention can also be resolved into their optical antipodes, e.g. by fractional crystallization. The compounds of the present invention may also be resolved by the formation of diastereomeric derivatives. Additional methods for the resolution of optical isomers, known to those skilled in the art, may be used. Such methods include those discussed by J. Jaques, A. Collet and S. Wilen in "Enantiomers, Racemates, and Resolutions", John Wiley and Sons, New York (1981). Optically active compounds can also be prepared from optically active starting materials, or by stereoselective synthesis.

Furthermore, when a double bond or a fully or partially saturated ring system is present in the molecule geometric isomers may be formed. It is intended that any geometric isomers, as separated, pure or partially purified geometric isomers or mixtures thereof are included within the scope of the invention. Likewise, molecules having a bond with restricted rotation may form geometric isomers. These are also intended to be included within the scope of the present invention.

Furthermore, some of the compounds of the present invention may exist in different tautomeric forms and it is intended that any tautomeric forms that the compounds are able to form are included within the scope of the present invention.

The invention also encompasses prodrugs of the present compounds, which on administration undergo chemical conversion by metabolic processes before becoming pharmacologically active substances. In general, such prodrugs will be functional derivatives of the compounds of the general formula IV which are readily convertible in vivo into the required compound of the formula IV. Conventional procedures for the selection and preparation of suitable prodrug derivatives are described, for example, in "Design of Prodrugs", ed. H. Bundgaard, Elsevier, 1985.

The invention also encompasses active metabolites of the present compounds.

Some compounds according to the invention inhibit the serotonin transporter and are thus serotonin reuptake inhibitors. Typically, the compounds have an in vitro uptake inhibition (IC50) of 5 µM or less, typically of 1 µM or less, preferably less than 500nM or less than 100 nM or less than 50 nM, preferably as measured by the method described in Example 13 - Measurements of "[³H]-5-HT uptake into rat cortical synaptosomes".

Some compounds according to the invention inhibit the norepinephrine transporter and are thus norepinephrine reuptake inhibitors. The compounds typically have an in vitro uptake inhibition (IC50) of 5 µM or less, typically of 1 µM or less, preferably less than 500nM, less than 100 nM or less than 50 nM, as measured by the method described in Example 13 - Measurements of "[³H]noradrenaline uptake into rat cortical synaptosomes".

Some compounds according to the invention inhibit the dopamine transporter and are thus dopamine reuptake inhibitors. Typically, such compounds have an in vitro uptake inhibition (IC50) of 5 µM or less, typically of 1 µM or less, preferably less than 500nM, less than 100 nM or less than 50 nM, preferably as measured by the method described in Example 13 - "Measurements of [³H]dopamine uptake into rat cortical synaptosomes".

As already mentioned, the compounds according to the invention are serotonin reuptake inhibitors and-they are thus considered to be applicable in the treatment of one or more of the following diseases/disorders: affective disorders, pain disorders, ADHD and stress urinary incontinence.

An embodiment concerns compounds of the invention having dual action, said compounds being serotonin reuptake inhibitors and norepinephrine reuptake inhibitors at the same time. Typically, such compounds have an in vitro uptake inhibition for the serotonin transporter which is at least 1, typically at least 5 or even more typically at least 10, 20 or 30 times higher than the in vitro uptake inhibition for the norepinephrine transporter as measured by the methods described in Example 13 - "Measurements of [³H]-5-HT uptake into rat cortical synaptosomes" and "Measurements of [³H]noradrenaline uptake into rat cortical synaptosomes".

An embodiment concerns compounds of the invention having dual action, said compounds being serotonin reuptake inhibitors and dopamine reuptake inhibitors at the same time. Typically, such compounds have an in vitro uptake inhibition for the serotonin transporter which is at least 1, typically at least 5 or even more typically at least 10, 20 or 30 times higher than the in vitro uptake inhibition for the dopamine transporter as measured by the methods described in Example 13 - "Measurements of [³H]-5-HT uptake into rat cortical synaptosomes" and "Measurements of [³H]dopamine uptake into rat cortical synaptosomes".

A further embodiment concerns compounds of the invention having triple action and thus being serotonin reuptake inhibitors, norepinephrine reuptake inhibitors and dopamine reuptake inhibitors.

In a further aspect the invention provides a compound of formula IV as the free base or a salt thereof for use as a medicament.

An embodiment of the invention provides a pharmaceutical composition comprising a compound of formula IV as the free base or a salt thereof and at least one pharmaceutically acceptable carrier or diluent. The composition may comprise any one of the embodiments of formula IV described above.

A further embodiment of the invention relates to the use of a compound of formula IV as the free base or a salt thereof for the preparation of a pharmaceutical composition for the treatment of a disease or disorder wherein a serotonin reuptake inhibitor is beneficial. Such pharmaceutical composition may comprise any one of the embodiments of formula IV described above.

A further embodiment of the invention relates to the use of a compound of formula IV as the free base or a salt thereof for the preparation of a pharmaceutical composition for the treatment of a disease or disorder, wherein a combined serotonin and norepinephrine reuptake inhibitor is beneficial. Such pharmaceutical composition may comprise any one of the embodiments of formula IV described above.

A further embodiment of the invention relates to the use of a compound of formula IV as the free base or a salt thereof for the preparation of a pharmaceutical composition for the treatment of a disease or disorder, wherein a combined serotonin and dopamine reuptake inhibitor is beneficial. Such pharmaceutical composition may comprise any one of the embodiments of formula IV described above.

A further embodiment of the present invention relates to the use of a compound of formula IV as the free base or a salt thereof for the preparation of a pharmaceutical composition for the treatment of a disease or disorder, wherein a combined serotonin, norepinephrine and dopamine reuptake inhibitor is beneficial. Such pharmaceutical composition may comprise any one of the embodiments of formula IV described above.

A further embodiment of the invention relates to the use of a compound of formula IV as the free base or a salt thereof for the preparation of a pharmaceutical composition for the treatment of affective disorders, pain disorders, ADHD and stress urinary incontinence.

In a further embodiment the present invention relates to the use of a compound of formula IV as the free base or a salt thereof for the preparation of a pharmaceutical composition for the treatment of affective disorders. To further illustrate without limiting the invention, the affective disorder to be treated is selected from the group consisting of depressive disorders and anxiety disorders:
A further embodiment concerns the use of a compound of formula IV as the free base or a salt thereof for the preparation of a pharmaceutical composition for the treatment of depressive disorders. Typically, the depressive disorder to be treated is selected from the group consisting of major depressive disorder, postnatal depression, dysthymia and depression associated with bipolar disorder, alzheimers, psychosis or parkinsons.

A further embodiment concerns the use of a compound of formula IV as the free base or a salt thereof for the preparation of a pharmaceutical composition for the treatment of anxiety disorders. Typically, the anxiety disorders to be treated are selected from the group consisting of general anxiety disorder, social anxiety disorder, post traumatic stress disorder, obsessive compulsive disorder, panic disorder, panic attacks, specific phobias, social phobia and agoraphobia.

In a further embodiment the present invention relates to the use of a compound of formula IV as the free base or a salt thereof for the preparation of a pharmaceutical composition for the treatment of pain disorders. To further illustrate without limiting the invention, the pain disorder to be treated is selected from the group consisting of fibromyalgia syndrome (FMS), overall pain, back pain, shoulder pain, headache as well as pain while awake and during daily activities.

In a further embodiment the present invention relates to the use of a compound of formula IV as the free base or a salt thereof for the preparation of a pharmaceutical composition for the treatment of attention deficit hyperactivity disorder.

In a further embodiment the present invention relates to the use of a compound of formula IV as the free base or a salt thereof for the preparation of a pharmaceutical composition for the treatment of stress urinary incontinence.

In a further aspect, the present invention relates to a method of preparing a compound of formula IV, comprising the nucleophilic substitution reaction of appropriately substituted indoles and appropriately substituted benzene sulfenyl chlorides.

The term "treatment" as used herein in connection with a disease or disorders includes also prevention as the case may be.

### PHARMACEUTICAL COMPOSITIONS

The present invention also relates to a pharmaceutical composition. The compounds of the invention may be administered alone or in combination with pharmaceutically acceptable carriers or diluents, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

The pharmaceutical compositions formed by combining the compound of the invention and the pharmaceutical acceptable carriers are then readily administered in a variety of dosage forms suitable for the disclosed routes of administration. The formulations may conveniently be presented in unit dosage form by methods known in the art of pharmacy.

The compounds of this invention are generally utilized as the free substance or as a pharmaceutically acceptable salt thereof. One example is an acid addition salt of a compound having the utility of a free base. When a compound of the invention contains a free base such salts are prepared in a conventional manner by treating a solution or suspension of a free base of the invention with a chemical equivalent of a pharmaceutically acceptable acid. Representative examples are mentioned above.

Pharmaceutical compositions for oral administration may be solid or liquid. Solid dosage forms for oral administration include e.g. capsules, tablets, dragees, pills, lozenges, powders, granules and tablette e.g. placed in a hard gelatine capsule in powder or pellet form or e.g. in the form of a troche or lozenge. Where appropriate, pharmaceutical compositions for oral administration may be prepared with coatings such as enteric coatings or they can be formulated so as to provide controlled release of the active ingredient such as sustained or prolonged release according to methods well known in the art. Liquid dosage forms for oral administration include e.g. solutions, emulsions, suspensions, syrups and elixirs.

Formulations of the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid, lower alkyl ethers of cellulose, corn starch, potato starch, gums and the like. Examples of liquid carriers are syrup, peanut oil, olive oil, phospho lipids, fatty acids, fatty acid amines, polyoxyethylene and water.

The carrier or diluent may include any sustained release material known in the art, such as glyceryl monostearate or glyceryl distearate, alone or mixed with a wax.

Any adjuvants or additives usually used for such purposes such as colourings, flavourings, preservatives etc. may be used provided that they are compatible with the active ingredients.

The amount of solid carrier may vary but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

Tablets may be prepared by mixing the active ingredient with ordinary adjuvants and/or diluents and subsequently compressing the mixture in a conventional tabletting machine.

Pharmaceutical compositions for parenteral administration include sterile aqueous and nonaqueous injectable solutions, dispersions, suspensions or emulsions as well as sterile powders to be reconstituted in sterile injectable solutions or dispersions prior to use. Depot injectable formulations are also contemplated as being within the scope of the present invention.

For parenteral administration, solutions of the compound of the invention in sterile aqueous solution, aqueous propylene glycol, aqueous vitamin E or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Solutions for injections may be prepared by dissolving the active ingredient and possible additives in a part of the solvent for injection, preferably sterile water, adjusting the solution to the desired volume, sterilising the solution and filling it in suitable ampoules or vials. Any suitable additive conventionally used in the art may be added, such as tonicity agents, preservatives, antioxidants, etc.

Other suitable administration forms include suppositories, sprays, ointments, cremes, gels, inhalants, dermal patches, implants, etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1, 2 or 3 dosages. The exact dosage will depend upon the frequency and mode of administration, the sex, age, weight and general condition of the subject treated, the nature and-severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1, 2 or 3 times per day may contain from 0.01 to about 1000 mg, such as about 0.01 to 100 mg, preferably from about 0.05 to about 500 mg, and more preferred from about 0.5 mg to about 200 mg.

For parenteral routes such as intravenous, intrathecal, intramuscular and similar administration, typically doses are in the order of about half the dose employed for oral administration.

Typical examples of recipes for the formulation of the invention are as follows:
1) Tablets containing 5.0 mg of a compound of the invention calculated as the free base:

| | |
|---|---|
| Compound of the invention | 5.0 mg |
| Lactose | 60 mg |
| Maize starch | 30 mg |
| Hydroxypropylcellulose | 2.4 mg |
| Microcrystalline cellulose | 19.2 mg |
| Croscarmellose Sodium Type A | 2.4 mg |
| Magnesium stearate | 0.84 mg |

2) Tablets containing 0.5 mg of a compound of the invention calculated as the free base:

| | |
|---|---|
| Compound of the invention | 0.5 mg |
| Lactose | 46.9 mg |
| Maize starch | 23.5 mg |
| Povidone | 1.8 mg |
| Microcrystalline cellulose | 14.4 mg |
| Croscarmellose Sodium Type A | 1.8 mg |
| Magnesium stearate | 0.63 mg |

3) Syrup containing per millilitre:

| | |
|---|---|
| Compound of the invention | 25 mg |
| Sorbitol | 500 mg |
| Hydroxypropylcellulose | 15 mg |
| Glycerol | 50 mg |
| Methyl-paraben | 1 mg |
| Propyl-paraben | 0.1 mg |
| Ethanol | 0.005 mL |
| Flavour | 0.05 mg |
| Saccharin sodium | 0.5 mg |
| Water | ad 1 mL |

4) Solution for injection containing per millilitre:

| | |
|---|---|
| Compound of the invention | 0.5 mg |
| Sorbitol | 5.1 mg |
| Acetic Acid | 0.05 mg |
| Saccharin sodium | 0.5 mg |
| Water | ad 1mL |

By the expression a compound of the invention is meant any one of the embodiments of formula IV as described herein.

In a further aspect the present invention relates to a method of preparing a compound of the invention as described in the following.

### METHODS OF PREPARATION OF THE COMPOUNDS OF THE INVENTION

### General Methods

Compounds of formula IV may be prepared by conventional synthetic techniques as described in the methods below.

### Method 1.

For the preparation of compounds of formula IV (for R¹, R² ≠ H). The appropriate indole of formula V is combined with the appropriate sulfenyl chloride of formula VI (for R¹, R² ≠ H) to generate the desired product of formula IV, using known methodology (Hamel P. et al. J. Heterocyclic Chem., 1999, 36, 643). The product of formula IV is isolated as the free base or a salt thereof. 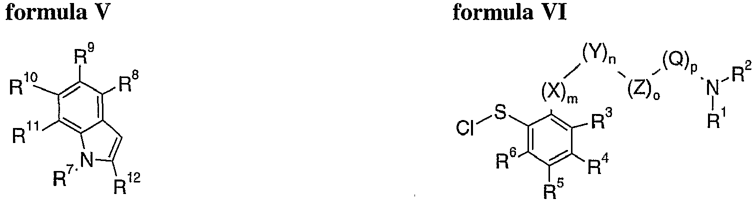 where R¹-R¹², X, Y, Z, Q, m, n, o, p are as defined herein.

Indoles of formula V are either commercially available or can be prepared according to methods described in standard works such as Houben-Weyl, Methoden der organischen Chemie (Methods of Organic Chemistry), Georg-Thieme-Verlag, Stuttgart; Organic Reactions, John Wiley & Sons, Inc. New York, namely under reaction conditions such as those which are known as suitable for such reactions.

### Method 2.

For the preparation of compounds of formula IV with R¹ = H. Compounds of formula VII are deprotected by standard techniques detailed in the textbook Protective Groups in Organic Synthesis Greene and Wuts, Wiley Interscience, (1999), ISBN 0471160199. The product of formula IV is isolated as the free base or a salt thereof. 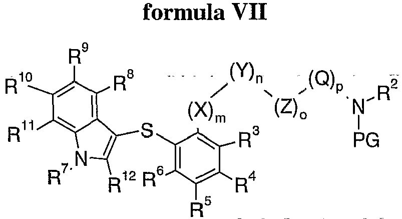 where R²-R¹², X, Y, Z, Q, m, n, o, p are as defined herein and PG is a nitrogen protecting group.

### Method 3.

For the preparation of compounds of formula IV (for R¹, R² ≠ H). Compounds of formula VIII are treated with a reducing agent such as *e.g*. LiAlH₄ or AlH₃. The product of formula IV is isolated as the free base or a salt thereof. 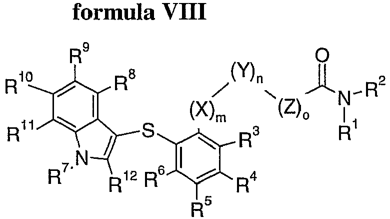 where R¹-R¹², X, Y, Z, m, n, o are as defined herein.

### Methods of preparation of intermediates for the synthesis of compounds of the invention

Intermediates for the synthesis of compounds of the invention may be prepared by conventional synthetic techniques as described in the methods below.

### Method 4.

For the preparation of compounds of formula VII: The appropriate indole of formula V is combined with the appropriate sulfenyl chloride of formula IX using known methodology (Hamel P. et al. J. Heterocyclic Chem., 1999, 36, 643). 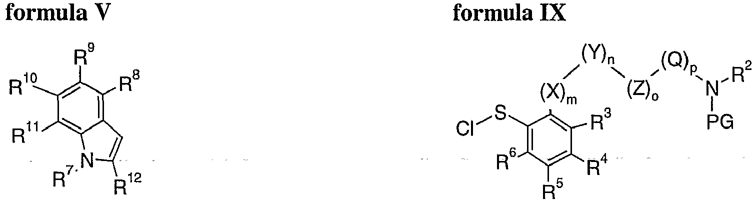 where R²-R¹², X, Y, Z, Q, m, n, o, p are as defined herein and PG is a nitrogen protecting group.

### Method 5.

For the preparation of compounds of formula VI (for R¹, R² ≠ H) and for the preparation of compounds of formula IX: Reaction of a thiophenol of formula X (for R¹, R² ≠ H) or of a thiophenol of formula XI with a chlorinating reagent such as *N*-chloro succinimide. 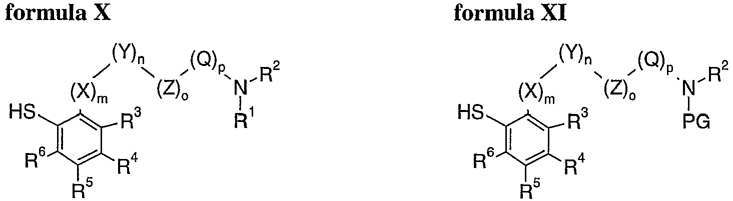 where R¹-R⁶, X, Y, Z, Q, m, n, o, p are as defined herein and PG is a nitrogen protecting group.

### Method 6.

For the preparation of compounds of formula X (for R¹, R² ≠ H) and of compounds of formula XI: Deprotection of the thiol moiety of a protected thiol of formula XII (for R¹, R² ≠ H) or of a protected thiol of formula XIII, by *e.g.* using a fluoride donor such as *e.g.* triethylamine tris(hydrogen fluoride). 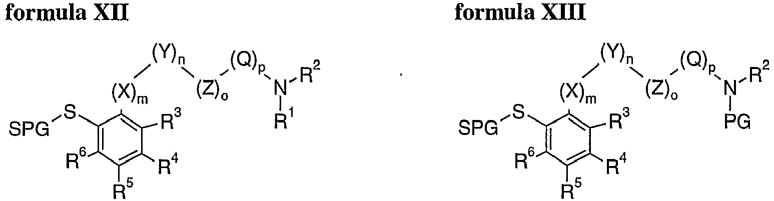 where R¹-R¹², X, Y, Z, Q, m, n, o, p are as defined herein, PG is a nitrogen protecting group and SPG is a thiol protecting group, *e.g.* a tri-*iso*-propyl silyl group.

### Method 7.

For the preparation of compounds of formula XII (for R¹, R² ≠ H) and compounds of formula XIII: Reaction of a compound of formula XIV (for R¹, R² ≠ H) or of a compound of formula XV with a protected thiol of formula XVI in the presence of a palladium catalyst and an appropriate base, according to Arnould, J. C. et al. Tetrahedron Letters, 1996, 37, 4523 and Winn M. et al. J. Med. Chem., 2001, 44, 4393. 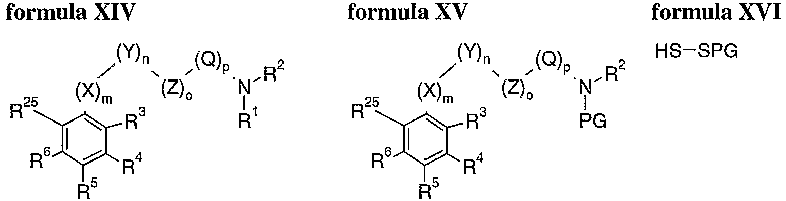 where R¹-R¹², X, Y, Z, Q, m, n, o, p are as defined herein, PG is a nitrogen protecting group, SPG is a thiol protecting group, *e.g.* a tri-*iso*-propyl silyl group and R²⁵ is a halogen such as iodine or bromine or R²⁵ is a pseudo halogen such as *e.g.* a trifluoro methyl sulphonyl group or a nonafluoro butyl sulphonyl group.

### Method 8.

For the preparation of compounds of formula XIV (for R¹, R² ≠ H; Q = CH₂) and for the preparation of compounds of formula XV (for Q = CH₂): Reduction of an amide of formula XVII (for R¹, R² ≠ H) or reduction of an amide of formula XVIII followed by protection of the nitrogen moiety with a nitrogen protecting group. 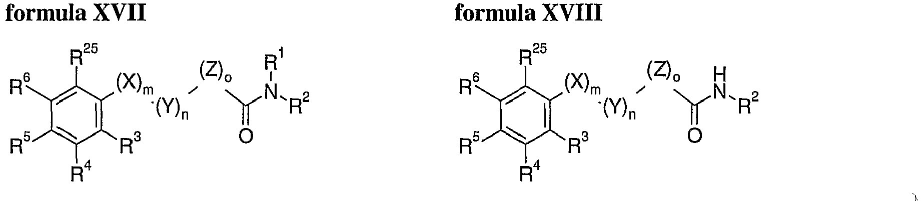 where R¹-R⁶, X, Y, Z, m, n, o are as defined herein and R²⁵ is a halogen such as iodine or bromine or R²⁵ is a pseudo halogen such as *e.g.* a trifluoro methyl sulphonyl group or a nonafluoro butyl sulphonyl group.

### Method 9.

For the preparation of compounds of formula XVII and for the preparation of compounds of formula XVIII: Activation of a carboxylic acid of formula XIX with an activating reagent such as *e*.*g*. Thionyl chloride, -*N*,*N*'-dicyclohexylcarbodiimide or carbonyl diimidazole followed by reaction with an amine of formula XX. 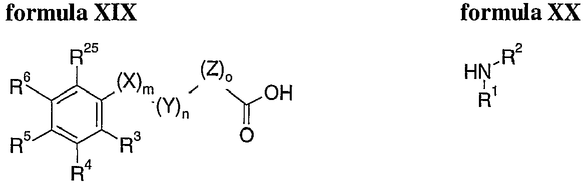 where R¹-R⁶, X, Y, Z, m, n, o are as defined herein and R²⁵ is a halogen such as iodine or bromine or R²⁵ is a pseudo halogen such as *e.g.* a trifluoro methyl sulphonyl group or a nonafluoro butyl sulphonyl group. Amines of formula XX are commercially available or can be prepared according to methods described in standard works such as Houben-Weyl, Methoden der organischen Chemie (Methods of Organic Chemistry), Georg-Thieme-Verlag, Stuttgart; Organic Reactions, John Wiley & Sons, Inc. New York, namely under reaction conditions such as those which are known as suitable for such reactions.

### Method 10.

For the preparation of compounds of formula XIV (for R¹, R² ≠ H; Q = CH₂ or CHR²²) and for the preparation of compounds of formula XV (for Q = CH₂ or CHR²²): Reductive amination of an aldehyde of formula XXI or of a ketone of formula XXII with an amine of formula XX, using a reducing reagent such as *e.g*. sodium cyanoborohydride. For R¹ = H, the reductive amination is followed by protection of nitrogen moiety with a nitrogen protecting group. 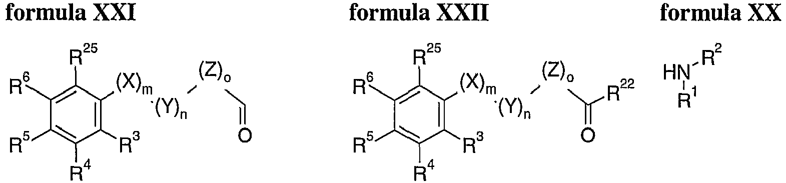 where R¹-R⁶, R²² X, Y, Z, m, n, o are as defined herein and R²⁵ is a halogen such as iodine or bromine or R²⁵ is a pseudo halogen such as *e.g.* a trifluoro methyl sulphonyl group or a nonafluoro butyl sulphonyl group.

### Method 11.

For the preparation of compounds of formula XXI (for n = 1, X = CH₂, Y = CH₂, CHR¹⁶) and for the preparation of compounds of formula XXII (for n = 1, X = CH₂, Y = CH₂, CHR¹⁶): A tandem Heck- isomerization reaction of a 1-bromo-2-iodobenzene compound of formula XXIII and an olefin of formula XXIV or of formula XXV according to Gibson et al. Synlett 1999, 954 and Qadir et al. Tetrahedron Letters, 44, 2003, 3675. 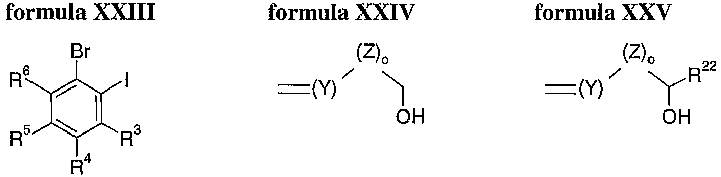 where R¹-R⁶, R²², Y, Z, o are as defined herein. 1-Bromo-2-iodobenzene compounds of formula XXIII, olefins of formula XXIV or of formula XXV are commercially available or can be prepared according to methods described in standard works such as Houben-Weyl, Methoden der organischen Chemie (Methods of Organic Chemistry), Georg-Thieme-Verlag, Stuttgart; Organic Reactions, John Wiley & Sons, Inc. New York, namely under reaction conditions such as those which are known as suitable for such reactions.

### Method 12.

For the preparation of compounds of formula VIII: Activation of a carboxylic acid of formula XXVI with an activating reagent such as *e.g*. carbonyl diimidazole followed by reaction with an amine of formula XX. 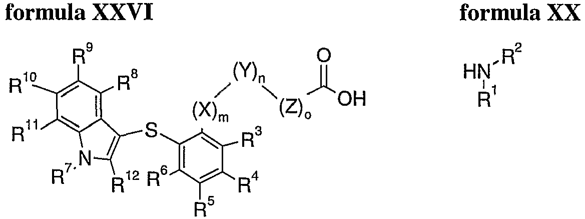 where R¹-R¹², X, Y, Z, m, n, o are as defined herein.

### Method 13.

For the preparation of compounds of formula XXVI: Hydrolysis of a carboxylic acid ester of formula XXVII. 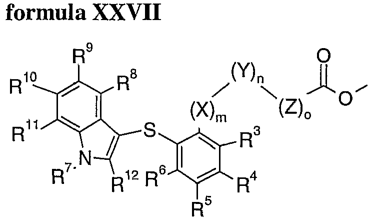 where R³-R¹², X, Y, Z, m, n, o are as defined herein.

### Method 14.

For the preparation of compounds of formula XXVII: The appropriate indole of formula V is combined with the appropriate sulfenyl chloride of formula XXVIII to generate desired product of formula XXVII, using known methodology (Hamel P. et al. J. Heterocyclic Chem., 1999, 36, 643). 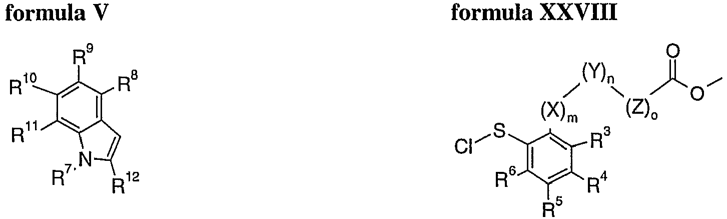 where R³-R¹², X, Y, Z, m, n, o are as defined herein.

### Method 15.

For the preparation of compounds of formula XXVIII: Reaction of a thiophenol of formula XXIX with a chlorinating reagent such as *N*-chloro succinimide. 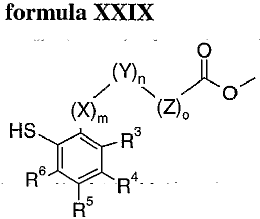 where R³-R⁶, X, Y, Z, m, n, o are as defined herein.

### Method 16.

For the preparation of compounds of formula XXIX: Deprotection of the thiol moiety of a protected thiol of formula XXX. 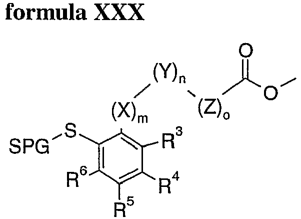 where R³-R⁶, X, Y, Z, m, n, o are as defined herein and SPG is a thiol protecting group, *e.g.* a tri-*iso*-propyl silyl group or a methoxycarbonyl ethyl group.

### Method 17.

For the preparation of compounds of formula XXX: Reaction of a compound of formula XXXI with a protected thiol of formula XVI in the presence of a palladium catalyst and an appropriate base according to Arnould, J. C. et al. Tetrahedron Letters, 1996, 37, 4523 and Winn M. et al. J. Med. Chem., 2001, 44, 4393. 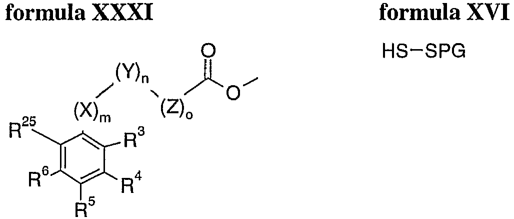 where R³-R⁶, X, Y, Z, m, n, o are as defined herein, SPG is a thiol protecting group, *e.g.* a tri-*iso-*propyl silyl group or a methoxycarbonyl ethyl group, and R²⁵ is a halogen such as iodine or bromine or R²⁵ is a pseudo halogen such as *e.g.* a trifluoro methyl sulphonyl group or a nonafluoro butyl sulphonyl group.

### Method 18.

For the preparation of compounds of formula XXXI: Fischer esterification of a carboxylic acid of formula XXXII: 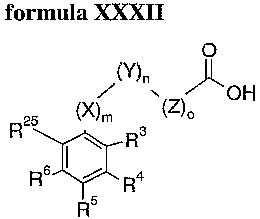 where R³-R⁶, X, Y, Z, m, n, o are as defined herein, and R²⁵ is a halogen such as iodine or bromine. Carboxylic acids of formula XXXII are commercially available or can be prepared according to methods described in standard works such as Houben-Weyl, Methoden der organischen Chemie (Methods of Organic Chemistry), Georg-Thieme-Verlag, Stuttgart; Organic Reactions, John Wiley & Sons, Inc. New York, namely under reaction conditions such as those which are known as suitable for such reactions.

### EXAMPLES

Analytical LC-MS data (Method A) were obtained on a PE Sciex API 150EX instrument equipped with atmospheric pressure photo ionisation and a Shimadzu LC-8A/SLC-10A LC system. Column: 30 X 4.6 mm Waters Symmetry C18 column with 3.5 µm particle size; Solventsystem: A = water/trifluoroacetic acid (100:0.05) and B = water/acetonitrile/trifluoroacetic acid (5:95:0.03); Method: Linear gradient elution with 90% A/10% B to 100% B in 4 minutes and with a flow rate of 2 mL/minute. Purity was determined by integration of the UV (254 nm) and ELSD trace. The retention times (t_{R}) are expressed in minutes.
Preparative LC-MS-purification was performed on the same instrument with atmospheric pressure chemical ionisation. Column: 50 X 20 mm YMC ODS-A with 5 µm particle size; Method: Linear gradient elution with 80% A to 100% B in 7 minutes and with a flow rate of 22.7 mL/minute. Fraction collection was performed by split-flow MS detection.

Analytical LC-MS-TOF (TOF = time of flight) data (Method B) were obtained on a micromass LCT 4-ways MUX equipped with a Waters 2488/Sedex 754 detector system. Column: 30 X 4.6 mm Waters Symmetry C18 column with 3.5 µm particle size; Solventsystem: A = water/trifluoroacetic acid (100:0.05) and B = water/acetonitrile/trifluoroacetic acid (5:95:0.03); Method: Linear gradient elution with 90% A/10% B to 100% B in 4 minutes and with a flow rate of 2 mL/minute. Purity was determined by integration of the UV (254 nm) and ELSD trace. The retention times (t_{R}) are expressed in minutes.

The invention disclosed herein is further illustrated by the following non-limiting examples.

### Preparation of the compounds of the invention

### Example 1

### Synthesis of 1. {2-[5-fluoro-2-(1H-indol-3-ylsulfanyl)-phenyl]-ethyl}-methyl-amine (Method 2)

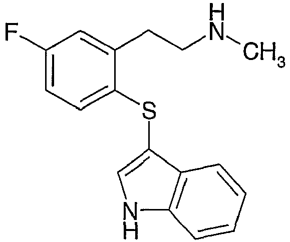

{2-[5-Fluoro-2-(1H-indol-3-ylsulfanyl)-phenyl]-ethyl}-methyl-carbamic acid tert-butyl ester (99 mg, 0.24 mmol) is dissolved in methanol (1.5 mL) and diethyl ether saturated with hydrochloric acid (0.5 mL) is added. The mixture is stirred at ambient temperature for 2 hours and concentrated *in vacuo.* Water (5 mL) is added to the remanence and the mixture is basified by addition of aqueous ammonia (25%). The aqueous fraction is extracted with ethyl acetate (3 x 10mL). The combined organic fractions are dried (MgSO₄) and concentrated *in vacuo.* The product is purified by preparative HPLC or silica gel chromatography eluting with ethyl acetate-ethanol-triethyl amine (100:5:5) to give 62 mg (83%) of the title compound.

The following compounds were prepared analogously:
2. {2-[2-(6-Fluoro-1H-indol-3-ylsulfanyl)-phenyl]-ethyl}-methyl-amine 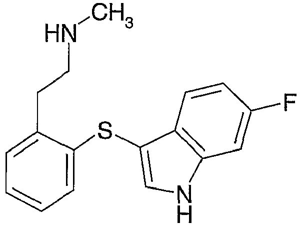
3. {2-[2-(5-Fluoro-1H-indol-3-ylsulfanyl)-phenyl]-ethyl}-methyl-amine 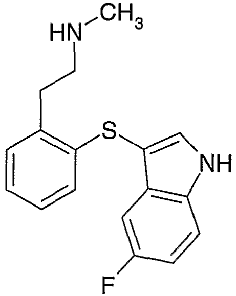
4. {2-[2-(4-Fluoro-1H-indol-3-ylsulfanyl)-phenyl]-ethyl}-methyl-amine 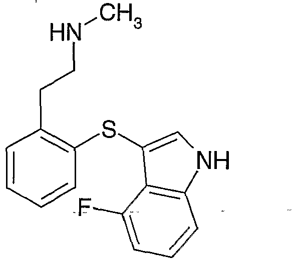
5. {2-[2-(7-Fluoro-1H-indol-3-ylsulfanyl)-phenyl]-ethyl}-methyl-amine 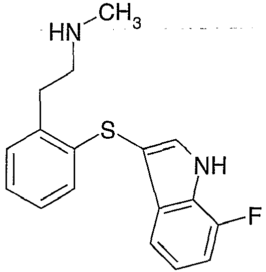
6. {2-[2-(7-Methoxy-1H-indol-3-ylsulfanyl)-phenyl]-ethyl}-methyl-amine 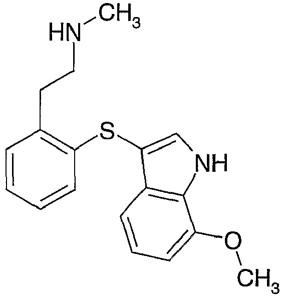
7. {2-[2-(5-Fluoro-2-methyl-1H-indol-3-ylsulfanyl)-phenyl]-ethyl}-methyl-amine 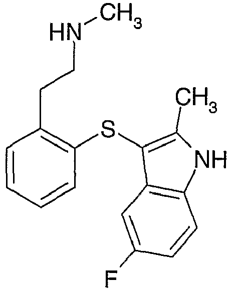
8. {2-[2-(5-Chloro-1H-indol-3-ylsulfanyl)-phenyl]-ethyl}-methyl-amine 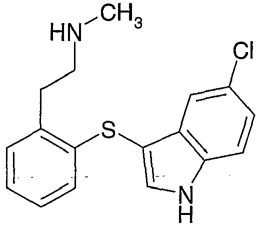
9. {2-[2-(4-Chloro-1H-indol-3-ylsulfanyl)-phenyl]-ethyl}-methyl-amine 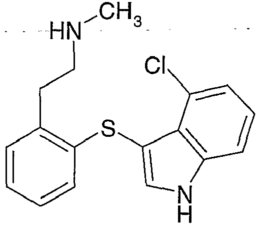
10. {2-[2-(7-Chloro-1H-indol-3-ylsulfanyl)-phenyl]-ethyl}-methyl-amine 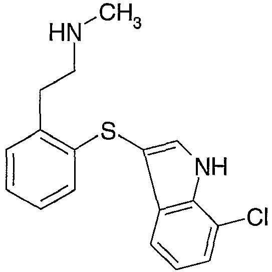
11. {2-[2-(1H-Indol-3-ylsulfanyl)-phenyl]-ethyl}-methyl-amine 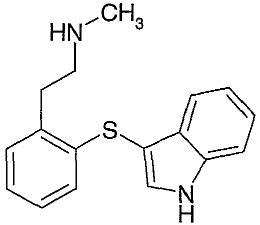
12. {2-[2-(1-Methyl-indol-3-ylsulfanyl)-phenyl]-ethyl}-methyl-amine 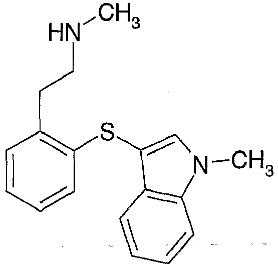
13. {2-[5-Chloro-2-(1H-indol-3-ylsulfanyl)-phenyl]-ethyl}-methyl-amine 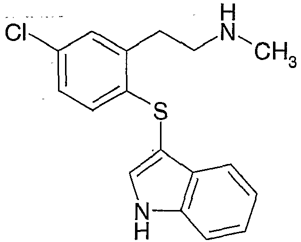
14. {2-[5-Chloro-2-(6-fluoro-1H-indol-3-ylsulfanyl)-phenyl]-ethyl}-methyl-amine 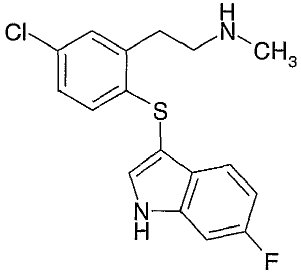
15. {2-[5-Chloro-2-(4-chloro-1H-indol-3-ylsulfanyl)-phenyl]-ethyl}-methyl-amine 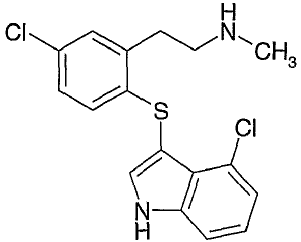
16. {2-[5-Fluoro-2-(6-fluoro-1H-indol-3-ylsulfanyl)-phenyl]-ethyl}-methyl-amine 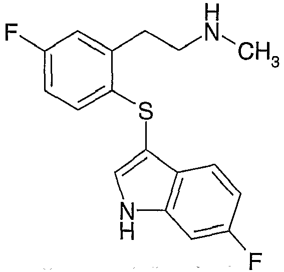
17. (2-(2-(4-Chloro-1H-indol-3-ylsulfanyl)-5-fluoro-phenyl)-ethyl)-methyl-amine 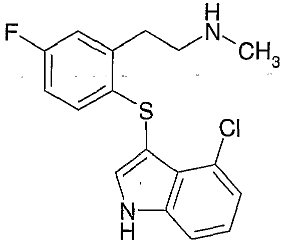
18. {2-[2-(6-Fluoro-1H-indol-3-ylsulfanyl)-4,5-dimethoxy-phenyl]-ethyl}-methyl-amine 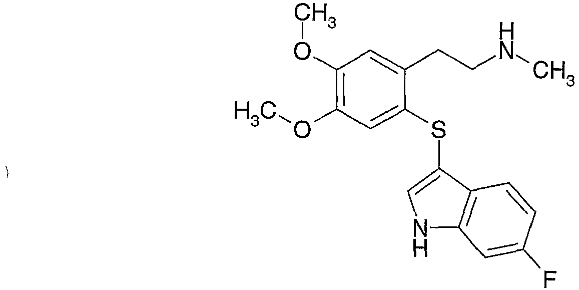
19. {2-[2-(4-Chloro-1H-indol-3-ylsulfanyl)-4,5-dimethoxy-phenyl]-ethyl}-methyl-amine 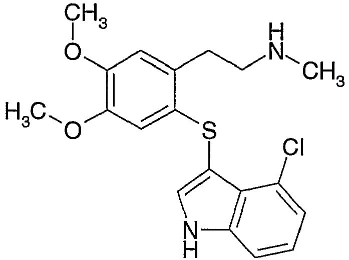
20. {2-[2-(1H-Indol-3-ylsulfanyl)-4,5-dimethoxy-phenyl]-ethyl}-methyl-amine 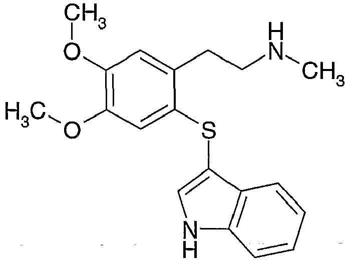
21. {4-[2-(1H-Indol-3-ylsulfanyl)-phenyl]-butyl}-methyl-amine 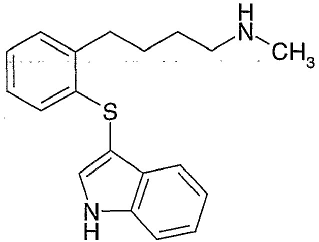
22. {4-[2-(4-Methoxy-1H-indol-3-ylsulfanyl)-phenyl]-butyl}-methyl-amine 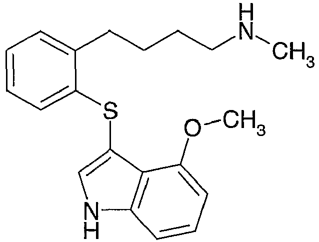
23. {4-[2-(4-Chloro-1H-indol-3-ylsulfanyl)-phenyl]-butyl}-methyl-amine 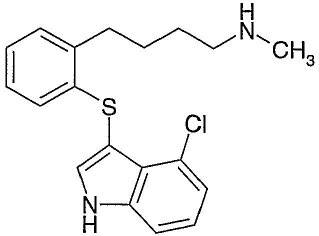
24. Methyl-{4-[2-(1-methyl-1H-indol-3-ylsulfanyl)-phenyl]-butyl}-amine

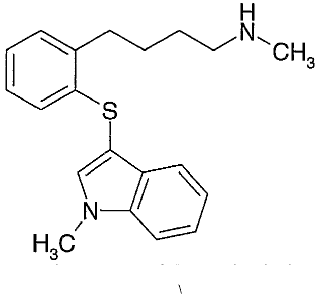

The following compounds were prepared analogously:
25. {3-[2-(4-Chloro-1H-indol-3-ylsulfanyl)-phenyl]-propyl}-methyl-amine 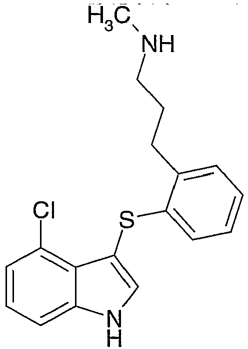
26. {3-[2-(4-Methoxy-1H-indol-3-ylsulfanyl)-phenyl]-propyl}-methyl-amine

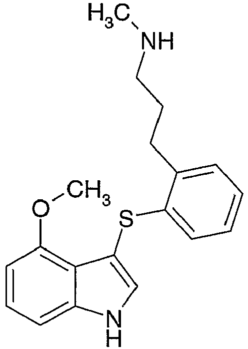

The following compound is prepared analogously:
27. Dimethyl-{3-[2-(3-methylamino-propyl)-phenylsulfanyl]-1H-indo1-5-yl}-amine 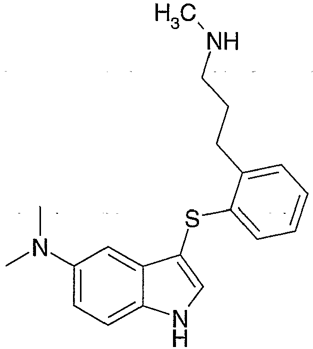
28. Methyl-{3-[2-(7-nitro-1H-indol-3-ylsulfanyl)-phenyl]-propyl}-amine

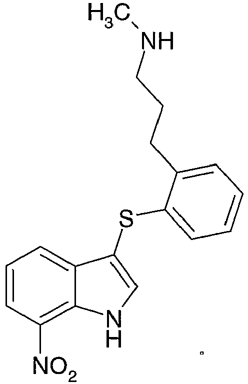

The following compounds were prepared analogously:
29. {3-[2-(6-Methanesulfonyl-1H-indol-3-ylsulfanyl)-phenyl]-propyl}-methyl-amine 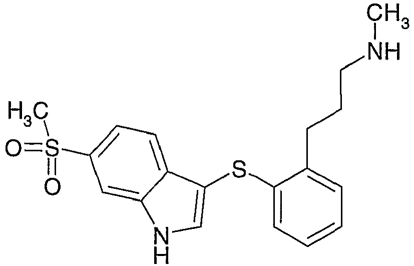
30. {3-[2-(1H-Indol-3-ylsulfanyl)-phenyl]-propyl}-methyl-amine 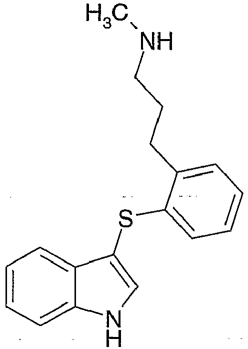
31. {3-[2-(6-Fluoro-1H-indol-3-ylsulfanyl)-phenyl]-propyl}-methyl-amine

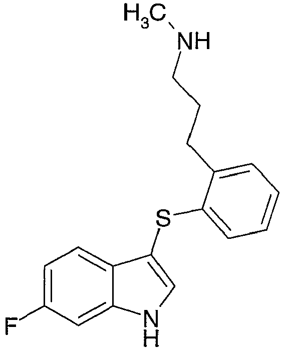

The following compound is prepared analogously:
32. Methyl-{3-[2-(1-methyl-1H-indol-3-ylsulfanyl)-phenyl]-propyl}-amine

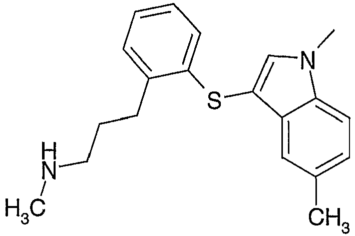

The following compound was prepared analogously:
33. Methyl-{3-[2-(5-methyl-1H-indol-3-ylsulfanyl)-phenyl]-propyl}-amine

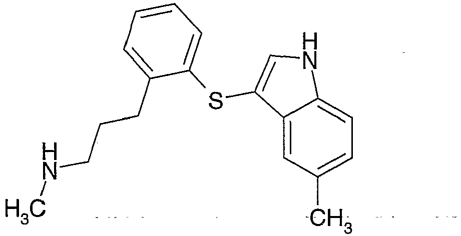

Analytical data of compounds 1-24 are shown in Table 2.

### Example 2

### Synthesis of 35. {2-[2-(1H-indol-3-ylsulfanyl)-phenyl]-ethyl}-dimethyl-amine (Method 3.)

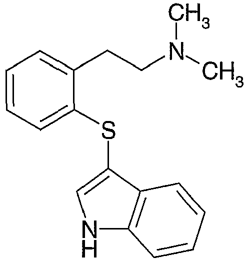

2-[2-(1H-Indol-3-ylsulfanyl)-phenyl]-*N,N-*dimethyl-acetamide (116 mg, 0.37 mmol) in 6 mL THF is added to LiAlH₄ (43 mg, 1.12 mmol) in 4 mL THF. The reaction mixture is stirred 16 hours at 50°C. The reaction is quenched with water and 2N NaOH. The reaction mixture is stirred for 1 hour, then 2.5 mL water is added and stirring was continued for another hour. The mixture is filtered, dried with MgSO₄ and concentrated *in vacuo.* Purification by flash chromatography or preparative HPLC to gives the title compound. The following compounds were prepared analogously:
36. 3-[2-(2-Morpholin-4-yl-ethyl)-phenylsulfanyl]-1H-indole 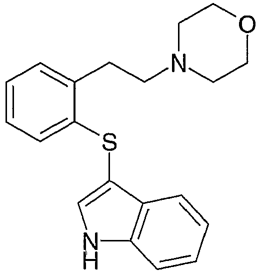
37. 3-[2-(2-Thiomorpholin-4-yl-ethyl)-phenylsulfanyl]-1H-indole

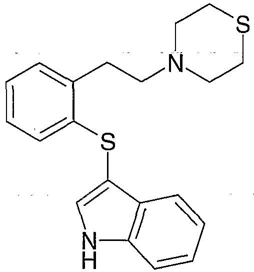

The following compound is prepared analogously:
34. 2-[2-(1H-Indol-3-ylsulfanyl)-phenyl]-ethylamine

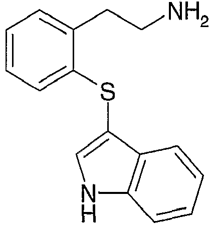

### Preparation of intermediates

### Example 3

### Synthesis of {2-[5-Fluoro-2-(1H-indol-3-ylsulfanyl)-phenyl]-ethyl}-methyl-carbamic acid tert-butyl ester (Method 4, Method 5, Method 14., Method 15.)

[2-(5-Fluoro-2-mercapto-phenyl)-ethyl]-methyl-carbamic acid tert-butyl ester (240 mg, 0.84 mmol) is dissolved in dry THF (3 mL) and added dropwise to a solution of N-chlorosuccinimide (112 mg, 0.84 mmol) in 1,2-dichloroethane (3 mL) at 0°C. The mixture is allowed to heat to ambient temperature and is stirred for 30 minutes. The resulting sulfenyl chloride solution is added dropwise to a solution of 1H-indole (147 mg, 1.26 mmol) in dry THF (3 mL) at 0°C. The mixture is stirred for 15 minutes at 0°C before adding saturated sodium bicarbonate solution (15 mL). The mixture is extracted with ethyl acetate (3 x 20 mL) and the combined organic fractions are washed with brine, dried (MgSO₄) and concentrated *in vacuo.* The product is purified by silica gel chromatography eluting with ethyl acetate-heptane, first (1:10) then (1:4). Upon evaporation of the volatiles 99 mg (29%) of the title compound is isolated.

The following intermediates were prepared analogously:
{2-[5-Fluoro-2-(1H-indol-3-ylsulfanyl)-phenyl]-ethyl}-methyl-carbamic acid tert-butyl ester
{2-[2-(6-Fluoro-1H-indol-3-ylsulfanyl)-phenyl]-ethyl}-methyl-carbamic acid tert-butyl ester
{2-[2-(5-Fluoro-1H-indol-3-ylsulfanyl)-phenyl]-ethyl}-methyl-carbamic acid tert-butyl ester
{2-[2-(4-Fluoro-1H-indol-3-ylsulfanyl)-phenyl]-ethyl}-methyl-carbamic acid tert-butyl ester
{2-[2-(7-Fluoro-1H-indol-3-ylsulfanyl)-phenyl]-ethyl}-methyl-carbamic acid tert-butyl ester
{2-[2-(7-Methoxy-1H-indol-3-ylsulfanyl)-phenyl]-ethyl}-methyl-carbamic acid tert-butyl ester
{2-[2-(5-Fluoro-2-methyl-1H-indol-3-ylsulfanyl)-phenyl]-ethyl}-methyl-carbamic acid tert-butyl ester
{2-[2-(5-Chloro-1H-indol-3-ylsulfanyl)-phenyl]-ethyl}-methyl-carbamic acid tert-butyl ester
{2-[2-(4-Chloro-1H-indol-3-ylsulfanyl)-phenyl]-ethyl}-methyl-carbamic acid tert-butyl ester
{2-[2-(7-Chloro-1H-indol-3-ylsulfanyl)-phenyl]-ethyl}-methyl-carbamic acid tert-butyl ester
{2-[2-(1H-Indol-3-ylsulfanyl)-phenyl]-ethyl}-methyl-carbamic acid tert-butyl ester
{2-[2-(1-Methyl-indol-3-ylsulfanyl)-phenyl]-ethyl}-methyl-carbamic acid tert-butyl ester
{2-[5-Chloro-2-(1H-indol-3-ylsulfanyl)-phenyl]-ethyl}-methyl-carbamic acid tert-butyl ester
{2-[5-Chloro-2-(6-fluoro-1H-indol-3-ylsulfanyl)-phenyl]-ethyl}-methyl-carbamic acid tert-butyl ester
{2-[5-Chloro-2-(4-chloro-1H-indol-3-ylsulfanyl)-phenyl]-ethyl}-methyl-carbamic acid tert-butyl ester
{2-[5-Fluoro-2-(6-fluoro-1H-indol-3-ylsulfanyl)-phenyl]-ethyl}-methyl-carbamic acid tert-butyl ester
(2-(2-(4-Chloro-1H-indol-3-ylsulfanyl)-5-fluoro-phenyl)-ethyl)-methyl-carbamic acid tert-butyl ester
{2-[2-(6-Fluoro-1H-indol-3-ylsulfanyl)-4,5-dimethoxy-phenyl]-ethyl}-methyl-carbamic acid tert-butyl ester
{2-[2-(4-Chloro-1H-indol-3-ylsulfanyl)-4,5-dimethoxy-phenyl]-ethyl}-methyl-carbamic acid tert-butyl ester
{2-[2-(1H-Indol-3-ylsulfanyl)-4,5-dimethoxy-phenyl]-ethyl}-methyl-carbamic acid tert-butyl-ester
{4-[2-(1H-Indol-3-ylsulfanyl)-phenyl]-butyl}-methyl-carbamic acid tert-butyl ester
4-[2-(4-Methoxy-1H-indol-3-ylsulfanyl)-phenyl]-butylcarbamic acid tert-butyl ester
{4-[2-(4-Chloro-1H-indol-3-ylsulfanyl)-phenyl]-butyl}-methyl-carbamic acid tert-butyl ester
Methyl-{4-[2-(1-methyl-1H-indol-3-ylsulfanyl)-phenyl]-butyl}-carbamic acid tert-butyl ester
[2-(1H-Indol-3-ylsulfanyl)-phenyl]-acetic acid methyl ester

The following intermediates are prepared analogously:
{3-[2-(4-Chloro-1H-indol-3-ylsulfanyl)-phenyl]-propyl}-methyl-carbamic acid tert-butyl ester
{3-[2-(4-Methoxy-1H-indol-3-ylsulfanyl)-phenyl]-propyl}-methyl-carbamic acid tert-butyl ester
{3-[2-(5-Dimethylamino-1H-indol-3-ylsulfanyl)-phenyl]-propyl}-methyl-carbamic acid tert-butyl ester
Methyl-{3-[2-(7-nitro-1H-indol-3-ylsulfanyl)-phenyl]-propyl}-carbamic acid tert-butyl ester
{3-[2-(6-Methanesulfonyl-1H-indol-3-ylsulfanyl)-phenyl]-propyl}-methyl-carbamic acid tert-butyl ester
{3-[2-(1H-Indol-3-ylsulfanyl)-phenyl]-propyl}-methyl-carbamic acid tert-butyl ester
{3-[2-(6-Fluoro-1H-indol-3-ylsulfanyl)-phenyl]-propyl}-methyl-carbamic acid tert-butyl ester
Methyl-{3-[2-(1-methyl-1H-indol-3-ylsulfanyl)-phenyl]-propyl}-carbamic acid tert-butyl ester
Methyl-{3-[2-(5-methyl-1H-indol-3-ylsulfanyl)-phenyl]-propyl}-carbamic acid tert-butyl ester

### Example 4

### Synthesis of [2-(5-fluoro-2-mercapto-phenyl)-ethyl]-methyl-carbamic acid tert-butyl ester (Method 6, Method 16.)

[2-(5-Fluoro-2-triisopropylsilanylsulfanyl-phenyl)-ethyl]-methyl-carbamic acid tert-butyl ester (1.60 g, 3.62 mmol) is dissolved in dry THF (12 mL) and triethylamine trihydrofluoride (0.59 g, 3.66 mmol) is added. The resulting mixture is heated at 60°C for 5 minutes, then cooled and concentrated in vacuo. The product is purified by eluting through a plug of silica with ethyl acetate-heptane (1:4) to give 0.72 g (72%) of the title compound as an oil.

The following intermediates were prepared analogously:
[2-(2-Mercapto-phenyl)-ethyl]-methyl-carbamic acid tert-butyl ester
[2-(5-Chloro-2-mercapto-phenyl)-ethyl]-methyl-carbamic acid tert-butyl ester
[2-(2-Mercapto-4,5-dimethoxy-phenyl)-ethyl]-methyl-carbamic acid tert-butyl ester
[4-(2-Mercapto-phenyl)-butyl]-methyl-carbamic acid tert-butyl ester
(2-Mercapto-phenyl)-acetic acid methyl ester
[3-(2-Mercapto-phenyl)-propyl]-methyl-carbamic acid tert-butyl ester

### Example 5

### Synthesis of [2-(5-fluoro-2-triisopropylsilanylsulfanyl-phenyl)-ethyl]-methyl-carbamic acid tert-butyl ester (Method 7, Method 17.)

[2-(2-Bromo-5-fluoro-phenyl)-ethyl]-methyl-carbamic acid tert-butyl ester (3.0 g, 9.03 mmol), tris(dibenzylideneacetone)dipalladium (0) (83 mg, 0.09 mmol), bis(2-diphenylphosphinophenyl)ether (97 mg, 0.18 mmol), sodium tert-butoxide (1.10 g, 11.7 mmol), triisopropylsilanethiol (1.90g, 9.93 mmol) and dry toluene (15 mL) are all placed in an Emrys Optimizer EXP 20 mL microwave reactor tube. The reaction vessel is sealed and subjected to microwave heating at 160°C for 15 minutes. Upon cooling the mixture is poured onto a plug of silica and the product is eluted with ethyl acetate-heptane (1:10). This furnishes 1.6 g (40%) of the title compound as an oil, which is used in the next step without further purification.

The following intermediates were prepared analogously:
Methyl-[2-(2-triisopropylsilanylsulfanyl-phenyl)-ethyl]-carbamic acid tert-butyl ester [2-(5-Chloro-2-triisopropylsilanylsulfanyl-phenyl)-ethyl]-methyl-carbamic acid tert-butyl ester
[2-(4,5-Dimethoxy-2-triisopropylsilanylsulfanyl-phenyl)-ethyl]-methyl-carbamic acid tert-butyl ester
Methyl-[4-(2-triisopropylsilanylsulfanyl-phenyl)-butyl]-carbamic acid tert-butyl ester (2-Triisopropylsilanylsulfanyl-phenyl)-acetic acid methyl ester
Methyl-[3-(2-triisopropylsilanylsulfanyl-phenyl)-propyl]-carbamic acid tert-butyl ester

### Example 6

### Synthesis of [2-(2-bromo-5-fluoro-phenyl)-ethyl]-methyl-amine (Method 8)

Lithium aluminum hydride (7.50 g, 197 mmol) is suspended in dry diethyl ether (75 mL) and cooled to 0°C. Aluminum (III) chloride (8.80g, 65.8 mmol) dissolved in dry diethyl ether (75 mL) is added dropwise at 0-5°C. The cooling bath is removed and the mixture is stirred at ambient temperature for 1 hour. The resulting aluminum hydride reagent solution is cooled to 0°C followed by dropwise addition of 2-(2-bromo-5-fluoro-phenyl)-N-methyl-acetamide (16.2 g, 65.8 mmol) dissolved in dry THF (150 mL). After complete addition the solution is allowed to heat to ambient temperature and stirring is continued for 16 hours.

The mixture is cooled to 10°C followed by slow dropwise addition of water (16 mL) followed by 2M sodium hydroxide (16 mL) and water (80 mL) to quench excessive reducing agent. The mixture is filtered and concentrated *in vacuo.* The remanence is redissolved in ethyl acetate (200 mL), dried (MgSO₄) and concentrated again to give 14.6 g (95%) of the title compound as an oil.

The following intermediates were prepared analogously:
[2-(2-Iodo-phenyl)-ethyl]-methyl-amine
[2-(2-Bromo-5-chloro-phenyl)-ethyl]-methyl-amine
[2-(2-Bromo-4,5-dimethoxy-phenyl)-ethyl]-methyl-amine
[4-(2-Bromo-phenyl)-butyl]-methyl-amine

### Synthesis of [2-(2-bromo-5-fluoro-phenyl)-ethyl]-methyl-carbamic acid tert-butyl ester (Method 8)

[2-(2-Bromo-5-fluoro-phenyl)-ethyl]-methyl-amine (14.6 g, 62.9 mmol) is dissolved in dry THF (200 mL) and di-tert-butyl dicarbonate (15.1 g, 69.2 mmol) is added. The mixture is stirred for 16 hours at ambient temperature. The volatiles are removed by means of evaporation and the crude mixture is purified by silica gel chromatography eluting with ethyl acetate-heptane (1:4) to furnish 19.4 g (93%) of the title compound as an oil.

The following intermediates were prepared analogously:
[2-(2-Iodo-phenyl)-ethyl]-methyl-carbamic acid tert-butyl ester
[2-(2-Bromo-5-chloro-phenyl)-ethyl]-methyl-carbamic acid tert-butyl ester
[2-(2-Bromo-4,5-dimethoxy-phenyl)-ethyl]-methyl-carbamic acid tert-butyl ester
[4-(2-Bromo-phenyl)-butyl]-methyl-carbamic acid tert-butyl ester

### Example 7

### Synthesis of 2-(2-bromo-5-fluoro-phenyl)-N-methyl-acetamide (Method 9)

Thionyl chloride (9.4 mL, 129 mmol) is added to a solution of (2-bromo-5-fluoro-phenyl)-acetic acid (20.0 g, 85.8 mmol) in dry toluene (400mL). The mixture is heated at reflux for 4 hours and the solvent is removed *in vacuo.* The remanence is redissolved in dry toluene (400 mL) and cooled to 0°C. 40% methylamine (aq.) (17.7 mL, 515 mmol) is added dropwise at 0-5°C. The mixture is then stirred at ambient temperature for 16 hours, poured onto water (250 mL) and extracted with ethyl acetate (3 x 200 mL). The combined organic fractions are washed successively with saturated sodium bicarbonate solution (150 mL) and brine (150 mL), dried (MgSO₄) and concentrated *in vacuo.* This gives 16.2 g (77%) of crystalline 2-(2-bromo-5-fluoro-phenyl)-N-methyl-acetamide.

The following intermediates were prepared analogously:
2-(2-Iodo-phenyl)-N-methyl-acetamide
2-(2-Bromo-5-chloro-phenyl)-N-methyl-acetamide
2-(2-Bromo-4,5-dimethoxy-phenyl)-N-methyl-acetamide
4-(2-Bromo-phenyl)-N-methyl-butyramide

### Example 8

### Synthesis of [3-(2-bromo-phenyl)-propyl]-methyl-carbamic acid tert-butyl ester (Method 10.)

Methyl amine (8M in ethanol, 38 mL, 304 mmol) is added to 3-(2-bromo-phenyl)-propionaldehyde (6.32 g, 29.7 mmol) and sodium cyanoborohydride (2.24 g, 35.6 mmol) in methanol. The reaction mixture is cooled to 0°C and acetic acid is added slowly until pH<7. The reaction mixture is stirred for ½ hour and neutralized with aqueous sodium hydroxide. Methanol is removed *in vacuo* and ethyl acetate and brine are added. The aqueous phase is extracted with ethyl acetate and the combined organic phases are dried with MgSO₄ and concentrated *in vacuo.* The residue is dissolved in THF (150 mL) and di-*tert*-butyl dicarbonate (7.2 g, 33 mmol) and triethyl amine (5.2 mL, 37.1 mmol) are added. The reaction mixture is stirred for 2 hours, filtered through silica gel and concentrated *in vacuo.* The residue is purified by flash chromatography (silica gel, ethyl acetate/heptane) to give 3.23 g (33%) of the title compound.

### Example 9

### Synthesis of 2-[2-(1H-indol-3-ylsulfanyl)-phenyl]-N,N-dimethyl-acetamide (Method 12.)

*N,N*'-Dicyclohexylcarbodiimide (875 mg, 4.2 mmol) is added to [2-(1H-Indol-3-ylsulfanyl)-phenyl]-acetic acid (600 mg, 2.1 mmol) in 3 mL dry DMF and 7 mL acetonitril and stirred for 10 minutes hours at room temperature. 5.3 mL dimethyl amine (2M in THF, 10.6 mmol) is added and the reaction mixture is stirred 16 hours at room temperature. The reaction mixture is concentrated *in vacuo.* The residue is extracted with ethyl acetate (3 times). The combined orgnic phases are washed with brine, dried with MgSO₄ and concentrated *in vacuo.* Purification by flash chromatography on silica gel (ethyl acetate/heptane) gives 116 mg (18%) of the title compound.

The following intermediates were prepared analogously:
2-[2-(1H-Indol-3-ylsulfanyl)-phenyl]-acetamide
2-[2-(1H-Indol-3-ylsulfanyl)-phenyl]-1-morpholin-4-yl-ethanone
2-[2-(1H-Indol-3-ylsulfanyl)-phenyl]-1-thiomorpholin-4-yl-ethanone

### Example 10

### Synthesis of [2-(1H-indol-3-ylsulfanyl)-phenyl]-acetic acid (Method 13.)

LiOH (1.6 g, 67.3 mmol) is added to [2-(1H-indol-3-ylsulfanyl)-phenyl]-acetic acid methyl ester (2 g, 6.73 mmol) in 27 mL mixture of THF/water (20:7). The reaction mixture is stirred for 10 minutes at 150°C in a Emrys Optimizer EXP microwave reactor under microwave heating and cooled to room temperature. The reaction mixture is poured into water and acidified with concentrated HCl. Ethyl acetate is added and the organic phase is washed with brine, dried with MgSO₄ and *concentrated in vacuo* to give the title compound, which is used in the next step without further purification.

### Example 11

### Synthesis of (2-bromo-phenyl)-acetic acid methyl ester (Method 18.)

2 mL H₂SO₄ is added to (2-bromo-phenyl)-acetic acid (15 g, 70 mmol) in 150 mL methanol. The reaction mixture is refluxed 16 hours and cooled to room temperature. 100 mL saturated NaHCO₃ (aq) is added. Methanol is removed *in vacuo.* The resulting mixture is extracted with Ethyl acetate. The organic phase is washed with brine, dried with MgSO₄ and concentrated *in vacuo* to give the title compound.

### Example 12

### Synthesis of 4-(2-bromo-phenyl)-butytic acid

Methanesulfonyl chloride (7.7 mL, 97 mmol) in 100 mL dry THF is added to a solution of 3-(2-bromo-phenyl)-propan-1-ol (17.4 g, 80.9 mmol) and triethyl amine (14.7 g, 146 mmol) in 200 mL dry THF at 0°C under an argon atmosphere. Water is added and the mixture is extracted with ethyl acetate. The organic phase is washed with brine, dried with MgSO₄ and concentrated *in vacuo* to give 23 g (97%) methanesulfonic acid 3-(2-bromo-phenyl)-propyl ester as an oil. Methanesulfonic acid 3-(2-bromo-phenyl)-propyl ester (23g, 78 mmol) in 300 mL dry DMF is added to a suspension of potassium cyanide (15.3 g, 235 mmol) in dry DMF. The reaction mixture is stirred at 60°C for 16 hours. Water is added and the mixture is extracted with ethyl acetate (3 times). The organic phase is washed with brine (twice), dried with MgSO₄ and *concentrated in vacuo.* The residue is placed on a plug of silica gel and eluted with ethyl acetate/heptane (1:4) and concentrated *in vacuo* to give 16.0 g 4-(2-bromo-phenyl)-butyronitrile (91%) as an oil. 300 mL concentrated HCl is added to 4-(2-bromo-phenyl)-butyronitrile (16.0 g, 71 mmol) in 150 mL acetic acid. The reaction mixture is stirred at 60°C for 16 hours. The reaction mixture concentrated *in vacuo* partly and is poured into water. The mixture is extracted with ethyl acetate (3 times). The organic phase is washed with brine (twice), dried with MgSO₄ and *concentrated in vacuo* to give the title compound as a crystalline material.

**Table 1 Reagents used for the preparation of compounds in Examples 1-10**

| **Name** | **Supplier** | **CAS no.** | **Cat.no.** |
|---|---|---|---|
| 6-Fluoroindole | Avocado | 399-51-9 | 24633 |
| 5-Fluoroindole | Aldrich | 399-52-0 | F910-8 |
| 4-Fluoroindole | Aldrich | 387-43-9 | 45,739-6 |
| 7-Fluoroindole | Lancaster | 387-44-0 | 17621 |
| 7-Methoxyindole | Aldrich | 3189-22-8 | 11,398-0 |
| 5-Fluoro-2-Methylindole | Aldrich | 399-72-4 | 51,153-6 |
| 5-Chloroindole | Aldrich | 17422-32-1 | C4,760-4 |
| 4-Chloroindole | Biosynth | 25235-85-2 | C4200 |
| 7-Chloroindole | Matrix | 53924-05-3 | 8757 |
| 1-Methylindole | Aldrich | 603-76-9 | 19,398-4 |
| 4-Methoxyindole | Biosynth | 4837-90-5 | M-3450 |
| 5-Aminoindole | | 5192-03-0 | |
| 7-Nitroindole | | 6960-42-5 | |
| 6-(Methylsulfonyl)-1h-Indole | Apollo | NA | OR7793 |
| 4-Methylindole | Acros | 16096-32-5 | 13389-0010 |
| Methylamine | Aldrich | 74-89-5 | 42,646-6 |
| Dimethylamine | Aldrich | 124-40-3 | 39,195-6 |
| Morpholine | Aldrich | 110-91-8 | 25,236-0 |
| Thiomorpholine | Fluka | 123-90-0 | 88885 |
| 2-Iodophenylacetic acid | Aldrich | 18698-96-9 | 53,147-2 |
| 2-Bromo-5-chlorophenylacetic acid | Apollo | 81682-38-4 | OR2153 |
| 2-Bromo-5-fluorophenylacetic acid | Matrix | NA | 11281 |
| 2-Bromo-4,5-dimethoxyphenylacetic acid | Apollo | 4697-62-5 | OR4518 |
| 3-(2-Bromophenyl)propionic acid | Transwld | 15115-58-9 | B3193 |
| 1-Bromo-2-iodobenzene | Aldrich | 583-55-1 | 24,261-6 |
| Allyl alcohol | Aldrich | 107-18-6 | 24,053-2 |
| 3-Buten-1-ol | Aldrich | 627-27-0 | 11,036-1 |
| N-Chlorosuccinimide | Aldrich | 128-09-6 | 10,968-1 |
| Sulfuryl Chloride | Aldrich | 7791-25-5 | 27,850-5 |
| Triisopropylsilanethiol | Aldrich | 156275-96-6 | 42,993-7 |
| Di*-tert-*butyl dicarbonate | Fluka | 24424-99-5 | 34660 |
| Bis(2-diphenylphosphinophenyl)ether | Aldrich | 166330-10-5 | 51,001-7 |
| Tris(dibenzylideneacetone)dipalladium (0) | Aldrich | 52409-22-0 | 32,877-4 |
| Sodium tert-butoxide | Aldrich | 865-48-5 | 35,927-0 |
| Triethylamine tris(hydrogen fluoride) | Aldrich | 73602-61-6 | 34,464-8 |
| Lithium aluminum hydride | Aldrich | 16853-85-3 | 21,277-6 |
| Aluminum chloride | Aldrich | 7446-70-0 | 29,471-3 |
| *N*,*N*'-Dicyclohexylcarbodiimide | Aldrich | 538-75-0 | D8,000-2 |
| 1,1'-Carbonyldiimidazole | Aldrich | 530-62-1 | 11,553-3 |
| Thionyl chloride | Acros | 7719-09-7 | 16949-0010 |

| | | | |
|---|---|---|---|
| NA: not available | | | |

**Table 2 Measured molecular mass (M+H⁺), measured HPLC-retention time (t_{R}, min) and UV- and ELSD-purities (%).**

| **Compound** | **LC/MS method** | **t_{R} min.** | **UV-purity (%)** | **ELSD-purity (%)** | **M+H⁺** |
|---|---|---|---|---|---|
| 1 | A | 1.87 | 93 | 99 | 301.0 |
| 2 | A | 1.90 | 99 | 99 | 302.1 |
| 3 | A | 1.87 | 98 | 99 | 301.1 |
| 4 | A | 1.80 | 97 | 99 | 301.0 |
| 5 | A | 1.88 | 94 | 100 | 301.1 |
| 6 | A | 1.86 | 92 | 100 | 313.2 |
| 7 | A | 1.92 | 99 | 100 | 315.0 |
| 8 | A | 1.99 | 98 | 100 | 317.1 |
| 9 | A | 1.89 | 100 | 100 | 317.1 |
| 10 | A | 2.01 | 96 | 100 | 317.1 |
| 11 | A | 1.81 | 99 | 100 | 283.2 |
| 12 | A | 2.00 | 99 | 99 | 297.1 |
| 13 | A | 2.00 | 98 | 99 | 317.1 |
| 14 | A | 2.08 | 97 | 98 | 334.9 |
| 15 | A | 2.07 | 95 | 97 | 351.1 |
| 16 | A | 1.96 | 98 | 96 | 318.9 |
| 17 | A | 1.94 | 98 | 98 | 335.1 |
| 18 | A | 1.80 | 97 | 99 | 361.1 |
| 19 | A | 1.83 | 99 | 98 | 377.1 |
| 20 | A | 1.74 | 96 | 99 | 342.9 |
| 21 | B | 1.68 | 100 | 100 | 311.2 |
| 22 | B | 1.62 | 92 | 100 | 341.3 |
| 23 | B | 1.70 | 97 | 100 | 345.2 |
| 24 | B | 1.85 | 97 | 100 | 325.3 |
| 25 | A* | 1.58 | 97 | 100 | 331.2 |
| 26 | A* | 1.47 | 91 | 100 | 327.3 |
| 29 | A** | 0.80 | 98 | 97 | 375.2 |
| 30 | A* | 1.51 | 96 | 100 | 297.3 |
| 31 | A* | 1.60 | 96 | 100 | 315.2 |
| 33 | A* | 1.64 | 95 | 100 | 311.4 |
| 35 | A* | 1.48 | 100 | 98 | 297.2 |
| 36 | A* | 1.48 | 99 | 99 | 339.3 |
| 37 | A* | 1.60 | 85 | 98 | 355.2 |

| | | | | | |
|---|---|---|---|---|---|
| *: same as method A, but with a column temperature of 40°C, **: same as method A, but but linear gradient elution with 90% eluent A/10% eluent B to 100% eluent B in 2.4 minutes, flow rate is 3.3 mL/minute and the coulumn is Waters SunFire (C18 3.5 µm 4.6 x 30 mm) | | | | | |

### Example 1

### Transporter inhibition assay

### Measurements of [³H]-5-HT uptake into rat cortical synaptosomes

Whole brains from male Wistar rats (125-225 g), excluding cerebellum, are homogenized in 0.40 M sucrose supplemented with 1mM nialamid with a glass/teflon homogenizer. The homogenate is centrifuged at 1000 x g for 10 min at 4 °C. The pellet is discarded and the supernatant is centrifuged at 40.000 x g for 20 min. The final pellet is homogenized in assay buffer (0.5 mg original tissue/well). Test compounds (or buffer) and 10 nM [³H]-5-HT are added to 96 well plates. Composition of assay buffer: 123 mM NaCl, 4.82 mM KCl, 0.973 mM CaCl₂, 1.12 mM MgSO₄, 12.66 mM Na₂HPO₄, 2.97 mM NaH₂PO₄, 0.162 mM EDTA, 2g/l glucose and 0,2g/l ascorbic acid. Buffer is oxygenated with 95% 0₂/5% CO₂ for 10 min. The incubation is started by adding tissue to a final assay volume of 0.2 mL. After 15 min incubation with radioligand at 37 °C, samples are filtered directly on Unifilter GF/C glass fiber filters (soaked for 30 min in 0.1% polyethylenimine) under vacuum and immediately washed with 1 x 0,2 ml assay buffer. Non-specific uptake is determined using citalopram (10 µM final concentration). Citalopram is included as reference in all experiments as dose-response curve.

### Measurements of [³H]noradrenaline uptake into rat cortical synaptosomes

Fresh occipital-, temporal- or parietal cortex from male Wistar rats (125-225 g) are homogenized in 0.4M sucrose with a glass/teflon homogenizer. The homogenate is centrifuged at 1000 x g for 10 min at 4 °C. The pellet is discarded and the supernatant is centrifuged at 40.000 x g for 20 min. The final pellet is homogenized in this assay buffer: 123 mM NaCl, 4.82 mM KCl, 0.973 mM CaCl₂, 1.12 mM MgSO₄, 12.66 mM Na₂HPO₄, 2.97 mM NaH₂PO₄, 0.162 mM EDTA, 2 g/l glucose and 0,2 g/l ascorbic acid (7,2 mg original tissue/mL = 1 mg/ 140µl). The buffer is oxygenated with 95% 0₂/5% CO₂ for 10 min. The pellet is suspended in 140 volumes of assaybuffer. Tissue is mixed with test compounds and after 10 min pre-incubation, 10 nM [³H]-noradrenaline is added to a final volume of 0,2 ml and the mixture is incubated for 15 min at 37°C. After 15 min incubation, samples are filtered directly on Unifilter GF/C glass fiber filters (soaked for 30 min in 0.1 % polyethylenimine) under vacuum and immediately washed with 1 x 0,2 mL assay buffer.

Non-specific uptake is determined using talsupram (10 µM final concentration). Duloxetine is included as reference in all experiments as dose-response curve.

### Measurements of [³H]dopamine uptake into rat cortical synaptosomes

Tissue preparation: male wistar rats (125-250 g) are sacrificed by decapitation and striatum quickly dissected out and placed in ice cold 0,40 M sucrose. The tissue is gently homogenised (glass teflon homogeniser) and the P2 fraction is obtained by centrifugation (1000 g, 10 minutes and 40000 g, 20 minutes, 4°C) and suspended in 560 volumes of a modified Krebs-Ringer-phosphate buffer, pH 7.4.

Tissue 0,25 mg/well(140µl) (original tissue) is mixed with test suspension. After 5 minutes pre-incubation at room temperature, 12.5 nM [³**H**]-dopamine is added and the mixture is incubated for 5 minutes at room temperature. Final volume is 0,2 mL.

The incubation is terminated by filtering the samples under vacuum through Whatman GF/C filters with a wash of 1 x 0,2ml buffer. The filters are dried and appropriate scintillation fluid (Optiphase Supermix) is added. After storage for 2 hours in the dark the content of radioactivity is determined by liquid scintillation counting. Uptake is obtained by subtracting the non-specific binding and passive transport measured in the presence of 100 µM of benztropin. For determination of the inhibition of uptake ten concentrations of drugs covering 6 decades are used.

³H-DA = 3,4-(ring-2,5,6-³H)dopamine hydrochloride from New England Nuclear, specific activity 30-50 Ci/mmol.
Hyttel, Biochem. Pharmacol. 1978, 27, 1063-1068;
Hyttel, Prog. Neuro-Psychopharmacol. & bil. Psychiat. 1982, 6, 277-295;
Hyttel & Larsen, Acta Pharmacol. Tox. 1985, 56, suppl. 1, 146-153.

## Claims

1. The present invention relates to a compound represented by the general formula IV 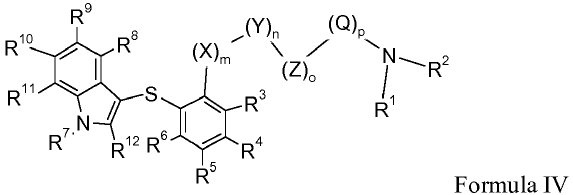 wherein
R¹-R² are independently selected from hydrogen, C₁₋₆-alk(en/yn)yl, C₃₋₈-cycloalk(en)yl, and C₃₋₈-cycloalk(en)yl-C₁₋₆-alk(en/yn)yl; or R¹ and R² together with the nitrogen form a 4-7 membered ring containing zero or one double bond, optionally said ring in addition to said nitrogen comprises one further heteroatom selected from oxygen and sulphur;
R³-R⁶ and R⁸-R¹² are independently selected from hydrogen, halogen, cyano, nitro, C₁₋₆-alk(en/yn)yl, C₃₋₈-cycloalk(en)yl, C₃₋₈-cycloalk(en)yl-C₁₋₆-alk(en/yn)yl, amino, C₁₋₆-alk(en/yn)ylamino, di-(C₁₋₆-alk(en/yn)yl)amino, C₁₋₆-alk(en/yn)ylcarbonyl, aminocarbonyl, C₁₋₆-alk(en/yn)ylaminocarbonyl, di-(C₁₋₆-alk(en/yn)yl)aminocarbonyl, hydroxy, C₁₋₆-alk(en/yn)yloxy, C₁₋₆-alk(en/yn)ylsulfanyl, halo-C₁₋₆-alk(en/yn)yl, halo-C₁₋₆-alk(en/yn)ylsulfonyl, halo-C₁₋₆-alk(en/yn)ylsulfanyl and C₁₋₆-alk(en/yn)ylsulfonyl;
R⁷ is selected from hydrogen, C₁₋₆-alk(en/yn)yl, C₃₋₈-cycloalk(en)yl and C₃₋₈-cycloalk(en)yl-C₁₋₆-alk(en/yn)yl;
X is selected from the group consisting of CH₂, CHR¹³ or CR¹⁴R¹⁵;
Y is selected from the group consisting of CH₂, CHR¹⁶ and CR¹⁷R¹⁸;
Z is selected from the group consisting of CH₂, CHR¹⁹ and CR²⁰R²¹
Q is selected from the group consisting of CH₂, CHR²² and CR²³R²⁴_{;} and
m, n, o and p are independently 0 or 1, wherein m+n+o+p equals to 1, 2, 3 or 4, with the proviso that when m+ n + o + p equals to 1 then none of X, Y, Z and Q are CH₂;
wherein R¹³- R²⁴ are independently selected from the group consisting of C₁₋₆-alk(en/yn)yl, C₃-₈-cycloalk(en)yl and C₃₋₈-cycloalk(en)yl-C₁₋₆-alk(en/yn)yl;
as the free base or a salt thereof

2. A compound according to claim 1 wherein R¹ and R² are independently selected from hydrogen and C₁₋₆-alk(en/yn)yl or wherein R¹ and R² together with the nitrogen form a 4-7 membered ring containing zero or one double bond, optionally said ring in addition to said nitrogen comprises one further heteroatom selected from oxygen and sulphur.

3. A compound according to any one of claims 1 and 2 wherein R³-R⁶ and R⁸-R¹² are independently selected from hydrogen, halogen, nitro, C₁₋₆-alk(en/yn)yl, di-(C₁₋₆-alk(en/yn)yl)amino, C₁₋₆-alk(en/yn)yloxy and C₁₋₆-alk(en/yn)ylsulfonyl.

4. A compound according to claim 3 wherein R³-R⁶ are independently selected from hydrogen, halogen and C₁₋₆-alk(en/yn)yloxy.

5. A compound according to claim 3 wherein R⁸-R¹² are independently selected from hydrogen, halogen, nitro, C₁₋₆-alk(en/yn)yl, di-(C₁₋₆-alk(en/yn)yl)amino, C₁₋₆-alk(en/yn)yloxy and C₁₋₆-alk(en/yn)ylsulfonyl.

6. A compound according to any one of claims 1-5 wherein R⁷ is selected from hydrogen and C₁₋₆-alk(en/yn)yl.

7. A compound according to any one of claims 1-6 wherein X, Y, Z and Q are CH₂.

8. A compound according to any one of claims 1-7, said compounds being selected from the following list of compounds
| **Compound No** | **Name** |
|---|---|
| 1 | {2-[5-Fluoro-2-(1H-indol-3-ylsulfanyl)-phenyl]-ethyl}-methyl-amine |
| 2 | {2-[2-(6-Fluoro-1H-indol-3-ylsulfanyl)-phenyl]-ethyl}-methyl-amine |
| 3 | {2-[2-(5-Fluoro-1H-indol-3-ylsulfanyl)-phenyl]-ethyl}-methyl-amine |
| 4 | {2-[2-(4-Fluoro-1H-indol-3-ylsulfanyl)-phenyl]-ethyl}-methyl-amine |
| 5 | {2-[2-(7-Fluoro-1H-indol-3-ylsulfanyl)-phenyl]-ethyl}-methyl-amine |
| 6 | {2-[2-(7-Methoxy-1H-indol-3-ylsulfanyl)-phenyl]-ethyl}-methyl-amine |
| 7 | {2-[2-(5-Fluoro-2-methyl-1H-indol-3-ylsulfanyl)-phenyl]-ethyl}-methyl-amine |
| 8 | {2-[2-(5-Chloro-1H-indol-3-ylsulfanyl)-phenyl]-ethyl}-methyl-amine |
| 9 | {2-[2-(4-Chloro-1H-indol-3-ylsulfanyl)-phenyl]-ethyl}-methyl-amine |
| 10 | {2-[2-(7-Chloro-1H-indol-3-ylsulfanyl)-phenyl]-ethyl}-methyl-amine |
| 11 | {2-[2-(1H-Indol-3-ylsulfanyl)-phenyl]-ethyl}-methyl-amine |
| 12 | {2-[2-(1-Methyl-indol-3-ylsulfanyl)-phenyl]-ethyl}-methyl-amine |
| 13 | {2-[5-Chloro-2-(1H-indol-3-ylsulfanyl)-phenyl]-ethyl}-methyl-amine |
| 14 | {2-[5-Chloro-2-(6-fluoro-1H-indol-3-ylsulfanyl)-phenyl]-ethyl}-methyl-amine |
| 15 | {2-[5-Chloro-2-(4-chloro-1H-indol-3-ylsulfanyl)-phenyl]-ethyl}-methyl-amine |
| 16 | {2-[5-Fluoro-2-(6-fluoro-1H-indol-3-ylsulfanyl)-phenyl]-ethyl}-methyl-amine |
| 17 | (2-(2-(4-Chloro-1H-indol-3-ylsulfanyl)-5-fluoro-phenyl)-ethyl)-methyl-amine |
| 18 | {2-[2-(6-Fluoro-1H-indol-3-ylsulfanyl)-4,5-dimethoxy-phenyl]-ethyl}-methyl-amine |
| 19 | {2-[2-(4-Chloro-1H-indol-3-ylsulfanyl)-4,5-dimethoxy-phenyl]-ethyl}-methyl-amine |
| 20 | {2-[2-(1H-Indol-3-ylsulfanyl)-4,5-dimethoxy-phenyl]-ethyl}-methyl-amine |
| 21 | {4-[2-(1H-Indol-3-ylsulfanyl)-phenyl]-butyl}-methyl-amine |
| 22 | {4-[2-(4-Methoxy-1H-indol-3-ylsulfanyl)-phenyl]-butyl}-methyl-amine |
| 23 | {4-[2-(4-Chloro-1H-indol-3-ylsulfanyl)-phenyl]-butyl}-methyl-amine |
| 24 | Methyl-{4-[2-(1-methyl-1H-indol-3-ylsulfanyl)-phenyl]-butyl} -amine |
| 25 | {3-[2-(4-Chloro-1H-indol-3-ylsulfanyl)-phenyl]-propyl}-methyl-amine |
| 26 | {3-[2-(4-Methoxy-1H-indol-3-ylsulfanyl)-phenyl]-propyl}-methyl-amine |
| 27 | Dimethyl-{3-[2-(3-methylamino-propyl)-phenylsulfanyl]-1H-indol-5-yl}-amine |
| 28 | Methyl-{3-[2-(7-nitro-1H-indol-3-ylsulfanyl)-phenyl]-propyl}-amine |
| 29 | {3-[2-(6-Methanesulfonyl-1H-indol-3-ylsulfanyl)-phenyl]-propyl}-methyl-amine |
| 30 | {3-[2-(1H-Indol-3-ylsulfanyl)-phenyl]-propyl}-methyl-amine |
| 31 | {3-[2-(6-Fluoro-1H-indol-3-ylsulfanyl)-phenyl]-propyl}-methyl-amine |
| 32 | Methyl-{3-[2-(1-methyl-1H-indol-3-ylsulfanyl)-phenyl]-propyl}-amine |
| 33 | Methyl-{3-[2-(5-methyl-1H-indol-3-ylsulfanyl)-phenyl]-propyl} -amine |
| 34 | 2-[2-(1H-Indol-3-ylsulfanyl)-phenyl]-ethylamine |
| 35 | {2-[2-(1H-Indol-3-ylsulfanyl)-phenyl]-ethyl}-dimethyl-amine |
| 36 | 3-[2-(2-Morpholin-4-yl-ethyl)-phenylsulfanyl]-1H-indole |
| 37 | 3-[2-(2-Thiomorpholin-4-yl-ethyl)-phenylsulfanyl]-1H-indole |
| | |
|---|---|
| as the free base or a salt thereof | |

9. A pharmaceutical composition comprising a compound according to any one of claims 1-8 and at least one pharmaceutically acceptable carrier or diluent.

10. A pharmaceutical composition according to claim 9 for use in the inhibition of the serotonin transporter.

11. A pharmaceutical composition according to claim 10 for use in the treatment of a disease or disorder wherein a serotonin reuptake inhibitor is beneficial.

12. A pharmaceutical composition according to claim 9 for use in the inhibition of the serotonin and noradrenaline transporters.

13. A pharmaceutical composition according to claim 12 for the treatment of a disease or disorder, wherein a combined serotonin and norepinephrine reuptake inhibitor is beneficial.

14. A pharmaceutical composition according to claim 9 for use in the inhibition of the serotonin and dopamine transporters.

15. A pharmaceutical composition according to claim 14 for use in the treatment of a disease or disorder, wherein a combined serotonin and dopamine reuptake inhibitor is beneficial.

16. A pharmaceutical composition according to claim 9 for use in the inhibition of the serotonin, noradrenaline and dopamine transporters.

17. A pharmaceutical composition according to claim 16 for use in the treatment of a disease or disorder, wherein a combined serotonin, norepinephrine and dopamine reuptake inhibitor is beneficial.

18. A pharmaceutical composition according to any one of claims 11, 13, 15 and 17, wherein said disorder or disease is an affective disorder.

19. A pharmaceutical composition according to claim 18, wherein the affective disorder is a depressive disorder such as major depressive disorder, postnatal depression, dysthymia or depression associated with bipolar disorder, alzheimers, psychosis or parkinsons.

20. A pharmaceutical composition according to claim 18, wherein the affective disorder is an anxiety disorder such as general anxiety disorder, social anxiety disorder, post traumatic stress disorder, obsessive compulsive disorder, panic disorder, panic attacks, specific phobias, social phobia and agoraphobia.

21. A pharmaceutical composition according to any one of claims 13 and 17, wherein said disorder or disease is a pain disorder such as fibromyalgia syndrome, overall pain, back pain, shoulder pain, headache as well as pain while awake and during daily activities.

22. A pharmaceutical composition according to any one of claims 13 and 17, wherein said disorder or disease is attention deficit hyperactivity disorder.

23. A pharmaceutical composition according to any one of claims 13 and 17, wherein said disorder or disease is stress urinary incontinence.

## Patentansprüche

1. Die vorliegende Erfindung bezieht sich auf eine Verbindung, die durch die allgemeine Formel IV dargestellt wird, 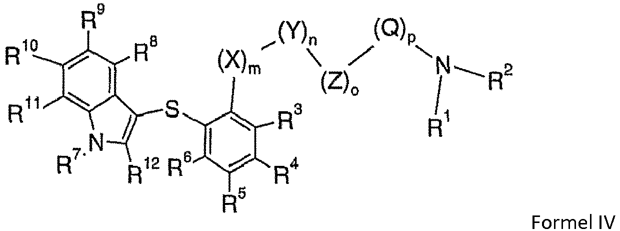 wobei
R¹ - R² unabhängig ausgewählt sind aus Wasserstoff, C₁₋₆-alk(en/yn)yl, C₃₋₈-cycloalk(en)yl, und C₃₋₈-cycloalk(en)yl-C₁₋₆-alk(en/yn)yl; oder R¹ und R² bilden zusammen mit dem Stickstoff einen 4 - 7 gliedrigen Ring, der Null oder eine Doppelbindung enthält, wobei gegebenenfalls der Ring zusätzlich zu dem Stickstoff ein weiteres Heteroatom umfasst, das ausgewählt ist aus Sauerstoff und Schwefel;
R³- R⁶ und R⁸ - R¹² unabhängig ausgewählt sind aus Wasserstoff, Halogen, Cyano, Nitro, C₁₋₆-alk(en/yn)yl, C₃₋₈-cycloalk(en)yl, C₃₋₈-cycloalk(en)yl-C₁₋₆-alk(en/yn)yl, Amino, C₁₋₆-alk(en/yn)ylamino, di-(C₁₋₆-alk(en/yn)yl)amino, C₁₋₆-alk(en/yn)ylcarbonyl, Aminocarbonyl, C₁₋₆-alk(en/yn)ylaminocarbonyl, di-(C₁₋₆-alk(en/yn)yl)aminocarbonyl, Hydroxy, C₁₋₆-alk(en/yn)yloxy, C₁₋₆-alk(en/yn)-ylsulfanyl, Halogen-C₁₋₆-alk/en/yn)yl, Halogen-C₁₋₆-alk(en/yn)ylsulfonyl, Halogen-C₁-₆-alk(en/yn)ylsulfanyl und C₁₋₆-alk(en/yn)ylsulfonyl;
R⁷ ausgewählt ist aus Wasserstoff, C₁₋₆-alk(en/yn)yl, C₃₋₈-cycloalk(en)yl und C₃₋₈-cycloalk(en)yl-C₁₋₆-alk(en/yn)yl;
X ausgewählt ist aus der Gruppe bestehend aus CH₂, CHR¹³ oder CR¹⁴R¹⁵;
Y ausgewählt ist aus der Gruppe bestehend aus CH₂, CHR¹⁶ und CR¹⁷R¹⁸;
Z ausgewählt ist aus der Gruppe bestehend aus CH₂, CHR¹⁹ und CR²⁰R²¹;
Q ausgewählt ist aus der Gruppe bestehend aus CH₂, CHR²² und CR²³R²⁴; und
m, n, o und p unabhängig 0 oder 1 sind, wobei m + n + o + p gleich 1, 2, 3 oder 4 ist, mit der Maßgabe, dass, wenn m + n + o + p gleich 1 ist, keines von X, Y, Z und Q CH₂ ist;
wobei R¹³ - R²⁴ unabhängig ausgewählt sind aus der Gruppe bestehend aus C₁₋₆-alk(en/yn)yl, C₃₋₈-cycloalk(en)yl und C₃₋₈-cycloalk(en)yl-C₁₋₆-alk(en/yn)yl;
als die freie Base oder ein Salz davon.

2. Verbindung nach Anspruch 1, wobei R¹ und R² unabhängig ausgewählt sind aus Wasserstoff und C₁₋₆-alk(en/yn)yl, oder wobei R¹ und R² zusammen mit dem Stickstoff einen 4 - 7 gliedrigen Ring bilden, der Null oder eine Doppelbindung enthält, wobei der Ring gegebenenfalls zusätzlich zu dem Stickstoff ein weiteres Heteroatom umfasst, das ausgewählt ist aus Sauerstoff und Schwefel.

3. Verbindung nach einem der Ansprüche 1 und 2, wobei R³ - R⁶ und R⁸- R¹² unabhängig ausgewählt sind aus Wasserstoff, Halogen, Nitro, C₁₋₆-alk(en/yn)yl, di-(C₁₋₆-alk(en/yn)yl)amino, C₁₋₆-alk(en/yn)yloxy und C₁₋₆-alk(en/yn)ylsulfonyl.

4. Verbindung nach Anspruch 3, wobei R³ - R⁶ unabhängig ausgewählt sind aus Wasserstoff, Halogen und C₁₋₆-alk(en/yn)yloxy.

5. Verbindung nach Anspruch 3, wobei R⁸ -R¹² unabhängig ausgewählt sind aus Wasserstoff, Halogen, Nitro, C₁₋₆-alk(en/yn)yl, di-(C₁₋₆-alk(en/yn)yl)amino, C₁₋₆-alk(en/yn)yloxy und C₁₋₆-alk(en/yn)ylsulfonyl.

6. Verbindung nach einem der Ansprüche 1 - 5, wobei R⁷ ausgewählt ist aus Wasserstoff und C₁₋₆-alk(en/yn)yl.

7. Verbindung nach einem der Ansprüche 1- 6, wobei X, Y, Z und Q CH₂ sind.

8. Verbindung nach einem der Ansprüche 1 - 7, wobei die Verbindungen ausgewählt sind aus der folgenden Liste von Verbindungen
| Verbindung Nr. | Name |
|---|---|
| 1 | {2-[5-Fluor-2-(1H-indol-3-ylsulfanyl)-phenyl]-ethyl}-methyl-amin |
| 2 | {2-[2-(6-Fluor-1H-indol-3-ylsulfanyl)-phenyl]-ethyl}-methyl-amin |
| 3 | {2-[2-(5-Fluor-1H-indol-3-ylsulfanyl)-phenyl]-ethyl}-methyl-amin |
| 4 | {2-[2-(4-Fluor-1H-indol-3-ylsulfanyl)-phenyl]-ethyl}-methyl-amin |
| 5 | {2-[2-(7-Fluor-1H-indol-3-ylsulfanyl)-phenyl]-ethyl}-methyl-amin |
| 6 | {2-[2-(7-Methoxy-1H-indol-3-ylsulfanyl)-phenyl]-ethyl}-methyl-amin |
| 7 | {2-[2-(5-Fluor-2-methyl-1H-indol-3-ylsulfanyl)-phenyl]-ethyl}-methyl-amin |
| 8 | {2-[2-(5-Chlor-1H-indol-3-ylsulfanyl)-phenyl]-ethyl}-methyl-amin |
| 9 | {2-[2-(4-Chlor-1H-indol-3-ylsulfanyl)-phenyl]-ethyl}-methyl-amin |
| 10 | {2-[2-(7-Chlor-1H-indol-3-ylsulfanyl)-phenyl]-ethyl}-methyl-amin |
| 11 | {2-[2-(1H-indol-3-ylsulfanyl)-phenyl]-ethyl}-methyl-amin |
| 12 | {2-[2-(1-Methyl-indol-3-ylsulfanyl)-phenyl]-ethyl}-methyl-amin |
| 13 | {2-[5-Chlor-2-(1H-indol-3-ylsulfanyl)-phenyl]-ethyl}-methyl-amin |
| 14 | {2-[5-Chlor-2-(6-fluor-1H-indol-3-ylsulfanyl)-phenyl]-ethyl}-methyl-amin |
| 15 | {2-[5-Chlor-2-(4-chlor-1H-indol-3-ylsulfanyl)-phenyl]-ethyl}-methyl-amin |
| 16 | {2-[5-Fluor-2-(6-fluor-1H-indol-3-ylsulfanyl)-phenyl]-ethyl}-methyl-amin |
| 17 | (2-(2-(4-Chlor-1H-indol-3-ylsulfanyl)-5-fluor-phenyl)-ethyl)-methyl-amin |
| 18 | {2-[2-(6-Fluor-1H-indol-3-ylsulfanyl)-4,5-dimethoxy-phenyl]-ethyl}-methyl-amin |
| 19 | {2-[2-(4-Chlor-1H-indol-3-ylsulfanyl)-4,5-dimethoxy-phenyl]-ethyl}-methyl-amin |
| 20 | {2-[2-(1H-Indol-3-ylsulfanyl)-4,5-dimethoxy-phenyl]-ethyl}-methyl-amin |
| 21 | {4-[2-(1H-Indol-3-ylsulfanyl)-phenyl]-butyl}-methyl-amin |
| 22 | {4-[2-(4-Methoxy-1H-indol-3-ylsulfanyl)-phenyl]-butyl}-methyl-amin |
| 23 | {4-[2-(4-Chlor-1H-indol-3-ylsulfanyl)-phenyl]-butyl}-methyl-amin |
| 24 | Methyl-{4-[2-(1-methyl-1H-indol-3-ylsulfanyl)-phenyl]-butyl}-amin |
| 25 | {3-[2-(4-Chlor-1H-indol-3-ylsulfanyl)-phenyl]-propyl}-methyl-amin |
| 26 | {3-[2-(4-Methoxy-1H-indol-3-ylsulfanyl)-phenyl]-propyl}-methyl-amin |
| 27 | Dimethyl-{3-[2-(3-methylamino-propyl)-phenylsulfanyl]-1H-indol-5-yl}-amin |
| 28 | Methyl-{3-[2-(7-nitro-1H-indol-3-ylsulfanyl)-phenyl]-propyl}-amin |
| 29 | {3-[2-(6-Methansulfonyl-1H-indol-3-ylsulfanyl)-phenyl]-propyl}-methyl-Amin |
| 30 | {3-[2-(1H-Indol-3-ylsulfanyl)-phenyl]-propyl}-methyl-amin |
| 31 | {3-[2-(6-Fluor-1H-indol-3-ylsulfanyl)-phenyl]-propyl}-methyl-amin |
| 32 | Methyl-{3-[2-(1-methyl-1H-indol-3-ylsulfanyl)-phenyl]-propyl}-amin |
| 33 | Methyl-{3-[2-(5-methyl-1H-indol-3-ylsulfanyl)-phenyl]-propyl}-amin |
| 34 | 2-[2-(1H-Indol-3-ylsulfanyl)-phenyl]-ethylamin |
| 35 | {2-[2-(1H-Indol-3-ylsulfanyl)-phenyl]-ethyl}-dimethyl-amin |
| 36 | 3-[2-(2-Morpholin-4-yl-ethyl)-phenylsulfanyl]-1H-indol |
| 37 | 3-[2-(2-Thiomorpholin-4-yl-ethyl)-phenylsulfanyl]-1H-indol |
als freie Base oder ein Salz davon.

9. Pharmazeutische Zusammensetzung, umfassend eine Verbindung nach einem der Ansprüche 1 - 8 und mindestens einen pharmazeutisch annehmbaren Träger oder ein Verdünnungsmittel.

10. Pharmazeutische Zusammensetzung nach Anspruch 9 zur Verwendung in der Hemmung des Serotonin-Transporters.

11. Pharmazeutische Zusammensetzung nach Anspruch 10 für die Verwendung in der Behandlung einer Krankheit oder Störung, wobei ein Serotonin-Wiederaufnahmehemmer nützlich ist.

12. Pharmazeutische Zusammensetzung nach Anspruch 9 für die Verwendung in der Hemmung der Serotonin- und Noradrenalin-Transporter.

13. Pharmazeutische Zusammensetzung nach Anspruch 12 für die Behandlung einer Krankheit oder Störung, wobei ein kombinierter Serotonin- und Norepinephrin-Wiederaufnahmehemmer nützlich ist.

14. Pharmazeutische Zusammensetzung nach Anspruch 9 zur Verwendung in der Hemmung der Serotonin- und Dopamin-Transporter.

15. Pharmazeutische Zusammensetzung nach Anspruch 14 zur Behandlung einer Krankheit oder Störung, wobei ein kombinierter Serotonin- und Dopamin-Wiederaufnahmehemmer nützlich ist.

16. Pharmazeutische Zusammensetzung nach Anspruch 9 zur Verwendung in der Hemmung der Serotonin-, Noradrenalin- und Dopamin-Transporter.

17. Pharmazeutische Zusammensetzung nach Anspruch 16 für die Behandlung einer Krankheit oder Störung, wobei ein kombinierter Serotonin-, Noradrenalin und Dopamin-Wiederaufnahmehemmer nützlich ist.

18. Pharmazeutische Zusammensetzung nach einem der Ansprüche 11, 13, 15 und 17, wobei die Störung oder Krankheit eine affektive Störung ist.

19. Pharmazeutische Zusammensetzung nach Anspruch 18, wobei die affektive Störung eine depressive Störung wie Major Depressive Disorder, postnatale Depression, Dysthymie oder eine Depression verbunden mit einer bipolaren Störung, Alzheimererkrankung, Psychose oder Parkinsonerkrankung ist.

20. Pharmazeutische Zusammensetzung nach Anspruch 18, wobei die affektive Störung eine Angststörung wie allgemeine Angststörung, soziale Angststörung, posttraumatische Belastungsstörung, Zwangsstörung, Panikstörung, Panikattacken, spezifische Phobien, soziale Phobie und Agoraphobie ist.

21. Pharmazeutische Zusammensetzung nach einem der Ansprüche 13 und 17, wobei die Störung oder Krankheit eine Schmerzkrankheit wie Fibromyalgie-Syndrom ist, allgemeine Schmerzen, Rückenschmerzen, Schulterschmerzen, Kopfschmerzen sowie Schmerzen im Wachzustand und während alltäglicher Aktivitäten ist.

22. Pharmazeutische Zusammensetzung nach einem der Ansprüche 13 und 17, wobei die Störung oder Krankheit eine Aufmerksamkeitsdefizit-Hyperaktivitätsstörung ist.

23. Pharmazeutische Zusammensetzung nach einem der Ansprüche 13 und 17, wobei die Störung oder Krankheit Stressharninkontinenz ist.

## Revendications

1. La présente invention concerne un composé représenté par la formule générale IV : 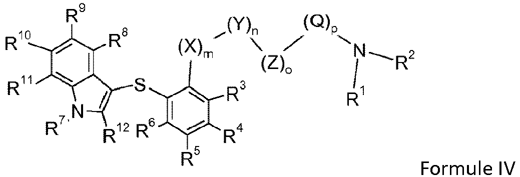 dans lequel :
R¹-R² sont indépendamment choisis parmi l'hydrogène, un alk(én/yn)yle en C₁₋₆, un cycloalk(én)yle en C₃₋₈ et un (cycloalk(én)yle en C₃₋₈)-(alk(én/yn)yle en C₁₋₆); ou R¹ et R² forment conjointement avec l'azote un noyau à 4 à 7 chaînons contenant zéro ou une double liaison, éventuellement ledit noyau comprend en plus de l'azote un autre hétéroatome choisi parmi l'oxygène et le soufre ;
R³-R⁶ et R⁸-R¹² sont indépendamment choisis parmi l'hydrogène, un halogène, un cyano, un nitro, un alk(én/yn)yle en C₁₋₆, un cycloalk(én)yle en C₃₋₈, un (cycloalk(én)yle en C₃₋₈]-(alk(én/yn)yle en C₁₋₆), un amino, un (alk(én/yn)yl en C₁₋₆)amino, un di-(alk(én/yn)yl en C₁₋₆)amino, un (alk(én/yn)yl en C₁₋₆)carbonyle, aminocarbonyle, (alk(én/yn)yl en C₁₋₆)aminocarbonyle, un di-(alk(én/yn)yl en C₁₋₆)aminocarbonyle, hydroxy, (alk(én/yn)yl en C₁₋₆)oxy, (alk(én/yn)yl en C₁₋₆)sulfanyle, un halo-alk(én/yn)yle en C₁₋₆, un halo-(alk(én/yn)yl en C₁₋₆)sulfonyle, un halo-(alk(én/yn)yl en C₁₋₆)sulfanyle et (alk(én/yn)yl en C₁₋₆)sulfonyle ;
R⁷ est choisi parmi l'hydrogène, un alk(én/yn)yle en C₁₋₆, un cycloalk(én)yle en C₃₋₈ et un (cycloalk(én)yle en C₃₋₈)-(alk(én/yn)yle en C₁₋₆) ;
X est choisi dans le groupe constitué de CH₂, CHR¹³ ou CR¹⁴R¹⁵;
Y est choisi dans le groupe constitué de CH₂, CHR¹⁶ et CR¹⁷R¹⁸;
Z est choisi dans le groupe constitué de CH₂, CHR¹⁹ et CR²⁰R²¹;
Q est choisi dans le groupe constitué de CH₂, CHR²² et CR²³R²⁴; et
m, n, o et p sont indépendamment 0 ou 1, dans lequel la somme m+n+o+p est égale à 1, 2, 3 ou 4 à condition que, lorsque la somme m+n+o+p est égale à 1, aucun de X, Y, Z et Q ne soit CH₂;
dans lequel R¹³-R²⁴ sont indépendamment choisis dans le groupe constitué d'un alk(én/yn)yle en C₁₋₆, d'un cycloalk(én)yle en C₃₋₈ et d'un (cycloalk(én)yle en C₃₋₈)-(alk(én/yn)yle en C₁₋₆);
sous la forme de la base libre ou d'un sel de celle-ci.

2. Composé selon la revendication 1, dans lequel R¹ et R² sont indépendamment choisis parmi l'hydrogène et un alk(én/yn)yle en C₁₋₆, ou dans lequel R¹ et R² forment conjointement avec l'azote un noyau à 4 à 7 chaînons contenant zéro ou une double liaison, éventuellement ledit noyau comprend en plus dudit azote un autre hétéroatome choisi parmi l'oxygène et le soufre.

3. Composé selon l'une quelconque des revendications 1 et 2, dans lequel R³-R⁶et R⁸-R¹² sont indépendamment choisi parmi l'hydrogène, un halogène, un nitro, un alk(én/yn)yle en C₁₋₆, un di-(alk(én/yn)yl en C₁₋₆)amino, un (alk(én/yn)yl en C₁₋₆)oxy et un (alk(én/yn)yl en C₁₋₆)sulfonyle.

4. Composé selon la revendication 3, dans lequel R³-R⁶ sont indépendamment choisis parmi l'hydrogène, un halogène et un (alk(én/yn)yl en C₁₋₆)oxy.

5. Composé selon la revendication 3, dans lequel R⁸-R¹² sont indépendamment choisi parmi l'hydrogène, un halogène, un nitro, un alk(én/yn)yle en C₁₋₆, un di-(alk(én/yn)yl en C₁₋₆)amino, un (alk(én/yn)yl en C₁₋₆)oxy et un (alk(én/yn)yl en C₁₋₆)sulfonyle.

6. Composé selon l'une quelconque des revendications 1 à 5, dans lequel R⁷ est choisi parmi l'hydrogène et un alk(én/yn)yle en C₁₋₆.

7. Composé selon l'une quelconque des revendications 1 à 6, dans lequel X, Y, Z et Q sont CH₂.

8. Composé selon l'une quelconque des revendications 1 à 7, lesdits composés étant choisis dans la liste suivante de composés :
| Composé | Nom |
|---|---|
| No | |
| 1 | {2-[5-Fluoro-2-(1H-indol-3-ylsulfanyl)phényl]éthyl}méthyl-amine |
| 2 | {2-[2-(6-Fluoro-1H-indol-3-ylsulfanyl)phényl]éthyl}méthyl-amine |
| 3 | {2-[2-(5-Fluoro-1H-indol-3-ylsulfanyl)phényl]éthyl}méthyl-amine |
| 4 | {2-[2-(4-Fluoro-1H-indol-3-ylsulfanyl)phényl]éthyl}méthyl-amine |
| 5 | {2-[2-(7-Fluoro-1H-indol-3-ylsulfanyl)phényl]éthyl}méthyl-amine |
| 6 | {2-[2-(7-Méthoxy-1H-indol-3-ylsulfanyl)phényl]éthyl}méthyl-amine |
| 7 | {2-[2-(5-Fluoro-2-méthyl-1H-indol-3-ylsulfanyl)phényl]éthyl}méthyl-amine |
| 8 | {2-[2-(5-Chloro-1H-indol-3-ylsulfanyl)phényl]éthyl}méthyl-amine |
| 9 | {2-[2-(4-Chloro-1H-indol-3-ylsulfanyl)phényl]éthyl}méthyl-amine |
| 10 | {2-[2-(7-Chloro-1H-indol-3-ylsulfanyl)phényl]éthyl}méthyl-amine |
| 11 | {2-[2-(1H-Indol-3-ylsulfanyl)phényl]éthyl}méthyl-amine |
| 12 | {2-[2-(1-Méthyl-indol-3-ylsulfanyl)phényl]éthyl}méthyl-amine |
| 13 | {2-[5-Chloro-2-(1H-indol-3-ylsulfanyl)phényl]éthyl}méthyl-amine |
| 14 | {2-[5-Chloro-2-(6-fluoro-1H-indol-3-ylsulfanyl)phényl]éthyl}méthyl-amine |
| 15 | {2-[5-Chloro-2-(4-chloro-1H-indol-3-ylsulfanyl)phényl]éthyl}méthyl-amine |
| 16 | {2-[5-Fluoro-2-(6-fluoro-1H-indol-3-ylsulfanyl)phényl]éthyl}méthyl-amine |
| 17 | (2-(2-(4-Chloro-1H-indol-3-ylsulfanyl)-5-fluoro-phényl)éthyl)méthyl-amine |
| 18 | {2-[2-(6-Fluoro-1H-indol-3-ylsulfanyl)-4,5-diméthoxy-phényl]éthyl}méthyl-amine |
| 19 | {2-[2-(4-Chloro-1H-indol-3-ylsulfanyl)-4,5-diméthoxy-phényl]éthyl}méthyl-amine |
| 20 | {2-[2-(1H-Indol-3-ylsulfanyl)-4,5-diméthoxy-phényl]éthyl}méthyl-amine |
| 21 | {4-[2-(1H-Indol-3-ylsulfanyl)phényl]butyl}méthyl-amine |
| 22 | {4-[2-(4-Méthoxy-1H-indol-3-ylsulfanyl)phényl]butyl}méthyl-amine |
| 23 | {4-[2-(4-Chloro-1H-indol-3-ylsulfanyl)phényl]butyl}méthyl-amine |
| 24 | Méthyl-{4-[2-(1-méthyl-1H-indol-3-ylsulfanyl)phényl]butyl}amine |
| 25 | {3-[2-(4-Chloro-1H-indol-3-ylsulfanyl)phényl]butyl}méthyl-amine |
| 26 | {3-[2-(4-Chloro-1H-indol-3-ylsulfanyl)phényl]propyl}méthyl-amine |
| 27 | Diméthyl-{3-[2-(3-méthylamino-propyl)phénylsulfanyl]-1H-indol-5-yl}amine |
| 28 | Méthyl-{3-[2-(7-nitro-1H-indol-3-ylsulfanyl)phényl]propyl}amine |
| 29 | {3-[2-(6-Méthanesulfonyl-1H-indol-3-ylsulfanyl)phényl]propyl}méthyl-amine |
| 30 | {3-[2-(1H-Indol-3-ylsulfanyl)phényl]propyl}méthyl-amine |
| 31 | {3-[2-(6-Fluoro-1H-indol-3-ylsulfanyl)phényl]propyl}méthyl-amine |
| 32 | Méthyl-{3-[2-(1-méthyl-1H-indol-3-ylsulfanyl)phényl]propyl}amine |
| 33 | Méthyl-{3-[2-(5-méthyl-1H-indol-3-ylsulfanyl)phényl]propyl}aminé |
| 34 | 2-[2-(1H-Indol-3-ylsulfanyl)phényl]éthylamine |
| 35 | {2-[2-(1H-Indol-3-ylsulfanyl)phényl]éthyl}diméthyl-amine |
| 36 | 3-[2-(2-Morpholin-4-yl-éthyl)phénylsulfanyl]-1H-indole |
| 37 | 3-[2-(2-Thiomorpholin-4-yl-éthyl)phénylsulfanyl]-1H-idole |
sous forme de base libre ou d'un de ses sels.

9. Composition pharmaceutique comprenant un composé selon l'une quelconque des revendications 1 à 8 et au moins un véhicule ou un diluant pharmaceutiquement acceptable.

10. Composition pharmaceutique selon la revendication 9 pour utilisation dans l'inhibition du transporteur de sérotonine.

11. Composition pharmaceutique selon la revendication 10 pour utilisation dans le traitement d'une maladie ou d'un trouble, dans laquelle un inhibiteur de recapture de sérotonine.

12. Composition pharmaceutique selon la revendication 9 pour utilisation dans l'inhibition des transporteurs de sérotonine et de noradrénaline.

13. Composition pharmaceutique selon la revendication 12 pour le traitement d'une maladie ou d'un trouble, dans laquelle un inhibiteur de recapture combiné de sérotonine et de norépinéphrine est bénéfique.

14. Composition pharmaceutique selon la revendication 9 pour utilisation dans l'inhibition des transporteurs de sérotonine et de dopamine.

15. Composition pharmaceutique selon la revendication 14 pour utilisation dans le traitement d'une maladie ou d'un trouble, dans laquelle un inhibiteur de recapture combiné de sérotonine et de dopamine est bénéfique.

16. Composition pharmaceutique selon la revendication 9 pour utilisation dans l'inhibition des transporteurs de sérotonine, de noradrénaline et de dopamine.

17. Composition pharmaceutique selon la revendication 16 pour utilisation dans le traitement d'une maladie ou d'un trouble, dans laquelle un inhibiteur de recapture combiné de sérotonine, de norépinéphrine et de dopamine est bénéfique.

18. Composition pharmaceutique selon l'une quelconque des revendications 11, 13, 15 et 17, dans laquelle ledit trouble ou ladite maladie est un trouble affectif.

19. Composition pharmaceutique selon la revendication 18, dans laquelle le trouble affectif est un trouble dépressif tel qu'un trouble dépressif majeur, une dépression post-natale, un état dépressif ou une dépression associée à un trouble bipolaire, à la maladie d'Alzheimer, à une psychose ou à la maladie de Parkinson.

20. Composition pharmaceutique selon la revendication 18, dans laquelle le trouble affectif est un trouble de l'anxiété tel qu'un trouble de l'anxiété généralisée, un trouble d'anxiété sociale, un trouble de stress post-traumatique, un trouble obsessif impulsif, un trouble de panique, des attaques de panique, des phobies spécifiques, une phobie sociale et une agoraphobie.

21. Composition pharmaceutique selon l'une quelconque des revendications 13 et 17, dans laquelle ledit trouble ou ladite maladie est un trouble de la douleur tel qu'un syndrome de fibromyalgie, une douleur globale, une douleur du dos, une douleur de l'épaule, un mal de tête ainsi qu'une douleur à l'état éveillé et au cours d'activités quotidiennes.

22. Composition pharmaceutique selon l'une quelconque des revendications 13 et 17, dans laquelle ledit trouble ou ladite maladie est un trouble d'hyperactivité déficitaire de l'attention.

23. Composition pharmaceutique selon l'une quelconque des revendications 13 et 17, dans laquelle ledit trouble ou ladite maladie est une incontinence urinaire à l'effort.
